(19) Europäisches Patentamt
European Patent Office
Office européen des brevets

(11) EP 2 481 749 A1

(12) **EUROPEAN PATENT APPLICATION**

(43) Date of publication:
**01.08.2012 Bulletin 2012/31**

(51) Int Cl.:
***C07K 14/47** (2006.01)* ***G01N 33/68** (2006.01)*

(21) Application number: **11193436.0**

(22) Date of filing: **25.11.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **25.11.2008 IT TO20080870**

(62) Document number(s) of the earlier application(s) in accordance with Art. 76 EPC:
**09796795.4 / 2 356 138**

(71) Applicant: **Bioindustry Park Silvano Fumero S.p.A.**
**10010 Colleretto Giacosa (IT)**

(72) Inventors:
- **Bucci, Enrico Maria**
  **10015 Ivrea (IT)**
- **Abrescia, Chiara**
  **10141 Torino (IT)**
- **Giuliano Albo, Alessandra**
  **10145 Torino (IT)**
- **Bongioanni, Paolo**
  **56124 Pisa (IT)**
- **Natale, Massimo**
  **10018 Pavone Canavese (IT)**
- **Corpillo, Davide**
  **10080 Pecco (IT)**
- **De Tata, Vincenzo**
  **56124 Pisa (IT)**
- **Franciosi, Lorenza**
  **13866 Masserano (IT)**
- **Lis, Katarzyna**
  **10015 Ivrea (IT)**

(74) Representative: **Jorio, Paolo et al**
**Studio Torta S.p.A.**
**Via Viotti, 9**
**10121 Torino (IT)**

Remarks:
This application was filed on 14-12-2011 as a divisional application to the application mentioned under INID code 62.

(54) **Biomarkers for diagnosing and detecting the progression of neurodegenerative disorders, in particular of amyotrophic lateral sclerosis**

(57) The present invention relates to biomarkers, to their use and to a method for detecting the progression of a neurodegenerative disease in an individual, in particular for Amyotrophic Lateral Sclerosis (ALS). The method comprises the steps of quantifying the level of one or more polypeptides in the biological sample according to the invention; comparing the obtained level with a reference level.

EP 2 481 749 A1

SEQUENCE LISTING

<110> Bioindustry Park del Canavese SPA

<120> BIOMARKERS FOR DIAGNOSING AND DETECTING THE PROGRESSION OF NEURODEGENERATIVE DISORDERS, IN PARTICULAR OF AMYOTROPHIC LATERAL SCLEROSIS

<130> 713-08

<160> 10

<170> PatentIn version 3.3

<210> 1
<211> 262
<212> PRT
<213> Homo sapiens

<220>
<223> polypeptide of spot 8 and 66

<400> 1

```
Asp Leu Gly Glu Glu Asn Phe Lys Ala Leu Val Leu Ile Ala Phe Ala
1               5                   10                  15

Gln Tyr Leu Gln Gln Cys Pro Phe Glu Asp His Val Lys Leu Val Asn
            20                  25                  30

Glu Val Thr Glu Phe Ala Lys Thr Cys Val Ala Asp Glu Ser Ala Glu
        35                  40                  45

Asn Cys Asp Lys Ser Leu His Thr Leu Phe Gly Asp Lys Leu Cys Thr
    50                  55                  60

Val Ala Thr Leu Arg Glu Thr Tyr Gly Glu Met Ala Asp Cys Cys Ala
65                  70                  75                  80

Lys Gln Glu Pro Glu Arg Asn Glu Cys Phe Leu Gln His Lys Asp Asp
                85                  90                  95

Asn Pro Asn Leu Pro Arg Leu Val Arg Pro Glu Val Asp Val Met Cys
            100                 105                 110

Thr Ala Phe His Asp Asn Glu Glu Thr Phe Leu Lys Lys Tyr Leu Tyr
        115                 120                 125

Glu Ile Ala Arg Arg His Pro Tyr Phe Tyr Ala Pro Glu Leu Leu Phe
    130                 135                 140

Phe Ala Lys Arg Tyr Lys Ala Ala Phe Thr Glu Cys Cys Gln Ala Ala
145                 150                 155                 160

Asp Lys Ala Ala Cys Leu Leu Pro Lys Leu Asp Glu Leu Arg Asp Glu
                165                 170                 175
```

```
Gly Lys Ala Ser Ser Ala Lys Gln Arg Leu Lys Cys Ala Ser Leu Gln
                180                 185                 190

Lys Phe Gly Glu Arg Ala Phe Lys Ala Trp Ala Val Ala Arg Leu Ser
            195                 200                 205

Gln Arg Phe Pro Lys Ala Glu Phe Ala Glu Val Ser Lys Leu Val Thr
    210                 215                 220

Asp Leu Thr Lys Val His Thr Glu Cys Cys His Gly Asp Leu Leu Glu
225                 230                 235                 240

Cys Ala Asp Asp Arg Ala Asp Leu Ala Lys Tyr Ile Cys Glu Asn Gln
                245                 250                 255

Asp Ser Ile Ser Ser Lys
                260

<210>  2
<211>  377
<212>  PRT
<213>  Homo sapiens

<220>
<223>  polypeptide of spot 110

<400>  2

Asp Leu Gly Glu Glu Asn Phe Lys Ala Leu Val Leu Ile Ala Phe Ala
1               5                   10                  15

Gln Tyr Leu Gln Gln Cys Pro Phe Glu Asp His Val Lys Leu Val Asn
                20                  25                  30

Glu Val Thr Glu Phe Ala Lys Thr Cys Val Ala Asp Glu Ser Ala Glu
            35                  40                  45

Asn Cys Asp Lys Ser Leu His Thr Leu Phe Gly Asp Lys Leu Cys Thr
    50                  55                  60

Val Ala Thr Leu Arg Glu Thr Tyr Gly Glu Met Ala Asp Cys Cys Ala
65                  70                  75                  80

Lys Gln Glu Pro Glu Arg Asn Glu Cys Phe Leu Gln His Lys Asp Asp
                85                  90                  95

Asn Pro Asn Leu Pro Arg Leu Val Arg Pro Glu Val Asp Val Met Cys
                100                 105                 110

Thr Ala Phe His Asp Asn Glu Glu Thr Phe Leu Lys Lys Tyr Leu Tyr
```

```
        115                 120                 125

Glu Ile Ala Arg Arg His Pro Tyr Phe Tyr Ala Pro Glu Leu Leu Phe
    130                 135                 140

Phe Ala Lys Arg Tyr Lys Ala Ala Phe Thr Glu Cys Cys Gln Ala Ala
145                 150                 155                 160

Asp Lys Ala Ala Cys Leu Leu Pro Lys Leu Asp Glu Leu Arg Asp Glu
                165                 170                 175

Gly Lys Ala Ser Ser Ala Lys Gln Arg Leu Lys Cys Ala Ser Leu Gln
            180                 185                 190

Lys Phe Gly Glu Arg Ala Phe Lys Ala Trp Ala Val Ala Arg Leu Ser
        195                 200                 205

Gln Arg Phe Pro Lys Ala Glu Phe Ala Glu Val Ser Lys Leu Val Thr
    210                 215                 220

Asp Leu Thr Lys Val His Thr Glu Cys Cys His Gly Asp Leu Leu Glu
225                 230                 235                 240

Cys Ala Asp Asp Arg Ala Asp Leu Ala Lys Tyr Ile Cys Glu Asn Gln
                245                 250                 255

Asp Ser Ile Ser Ser Lys Leu Lys Glu Cys Cys Glu Lys Pro Leu Leu
            260                 265                 270

Glu Lys Ser His Cys Ile Ala Glu Val Glu Asn Asp Glu Met Pro Ala
        275                 280                 285

Asp Leu Pro Ser Leu Ala Ala Asp Phe Val Glu Ser Lys Asp Val Cys
    290                 295                 300

Lys Asn Tyr Ala Glu Ala Lys Asp Val Phe Leu Gly Met Phe Leu Tyr
305                 310                 315                 320

Glu Tyr Ala Arg Arg His Pro Asp Tyr Ser Val Val Leu Leu Leu Arg
                325                 330                 335

Leu Ala Lys Thr Tyr Glu Thr Thr Leu Glu Lys Cys Cys Ala Ala Ala
            340                 345                 350

Asp Pro His Glu Cys Tyr Ala Lys Val Phe Asp Glu Phe Lys Pro Leu
        355                 360                 365

Val Glu Glu Pro Gln Asn Leu Ile Lys
    370                 375
```

<210> 3
<211> 274
<212> PRT
<213> Homo sapiens

<220>
<223> polypeptide of spot 65

<400> 3

```
Asp Leu Gly Glu Glu Asn Phe Lys Ala Leu Val Leu Ile Ala Phe Ala
1               5                   10                  15

Gln Tyr Leu Gln Gln Cys Pro Phe Glu Asp His Val Lys Leu Val Asn
            20                  25                  30

Glu Val Thr Glu Phe Ala Lys Thr Cys Val Ala Asp Glu Ser Ala Glu
        35                  40                  45

Asn Cys Asp Lys Ser Leu His Thr Leu Phe Gly Asp Lys Leu Cys Thr
    50                  55                  60

Val Ala Thr Leu Arg Glu Thr Tyr Gly Glu Met Ala Asp Cys Cys Ala
65                  70                  75                  80

Lys Gln Glu Pro Glu Arg Asn Glu Cys Phe Leu Gln His Lys Asp Asp
                85                  90                  95

Asn Pro Asn Leu Pro Arg Leu Val Arg Pro Glu Val Asp Val Met Cys
            100                 105                 110

Thr Ala Phe His Asp Asn Glu Glu Thr Phe Leu Lys Lys Tyr Leu Tyr
        115                 120                 125

Glu Ile Ala Arg Arg His Pro Tyr Phe Tyr Ala Pro Glu Leu Leu Phe
    130                 135                 140

Phe Ala Lys Arg Tyr Lys Ala Ala Phe Thr Glu Cys Cys Gln Ala Ala
145                 150                 155                 160

Asp Lys Ala Ala Cys Leu Leu Pro Lys Leu Asp Glu Leu Arg Asp Glu
                165                 170                 175

Gly Lys Ala Ser Ser Ala Lys Gln Arg Leu Lys Cys Ala Ser Leu Gln
            180                 185                 190

Lys Phe Gly Glu Arg Ala Phe Lys Ala Trp Ala Val Ala Arg Leu Ser
        195                 200                 205

Gln Arg Phe Pro Lys Ala Glu Phe Ala Glu Val Ser Lys Leu Val Thr
```

210 215 220

Asp Leu Thr Lys Val His Thr Glu Cys Cys His Gly Asp Leu Leu Glu
225 230 235 240

Cys Ala Asp Asp Arg Ala Asp Leu Ala Lys Tyr Ile Cys Glu Asn Gln
245 250 255

Asp Ser Ile Ser Ser Lys Leu Lys Glu Cys Cys Glu Lys Pro Leu Leu
260 265 270

Glu Lys

<210> 4
<211> 110
<212> PRT
<213> Homo sapiens

<220>
<223> polypeptide of spot 34

<400> 4

Cys Cys Thr Glu Ser Leu Val Asn Arg Arg Pro Cys Phe Ser Ala Leu
1 5 10 15

Glu Val Asp Glu Thr Tyr Val Pro Lys Glu Phe Asn Ala Glu Thr Phe
20 25 30

Thr Phe His Ala Asp Ile Cys Thr Leu Ser Glu Lys Glu Arg Gln Ile
35 40 45

Lys Lys Gln Thr Ala Leu Val Glu Leu Val Lys His Lys Pro Lys Ala
50 55 60

Thr Lys Glu Gln Leu Lys Ala Val Met Asp Asp Phe Ala Ala Phe Val
65 70 75 80

Glu Lys Cys Cys Lys Ala Asp Asp Lys Glu Thr Cys Phe Ala Glu Glu
85 90 95

Gly Lys Lys Leu Val Ala Ala Ser Gln Ala Ala Leu Gly Leu
100 105 110

<210> 5
<211> 194
<212> PRT
<213> Homo sapiens

<220>
<223> polypeptide of spot 125

<400> 5

```
Ala Ile Gln Leu Thr Tyr Asn Pro Asp Glu Ser Ser Lys Pro Asn Met
1               5                   10                  15

Ile Asp Ala Ala Thr Leu Lys Ser Arg Lys Met Leu Glu Glu Ile Met
            20                  25                  30

Lys Tyr Glu Ala Ser Ile Leu Thr His Asp Ser Ser Ile Arg Tyr Leu
        35                  40                  45

Gln Glu Ile Tyr Asn Ser Asn Asn Gln Lys Ile Val Asn Leu Lys Glu
    50                  55                  60

Lys Val Ala Gln Leu Glu Ala Gln Cys Gln Glu Pro Cys Lys Asp Thr
65                  70                  75                  80

Val Gln Ile His Asp Ile Thr Gly Lys Asp Cys Gln Asp Ile Ala Asn
                85                  90                  95

Lys Gly Ala Lys Gln Ser Gly Leu Tyr Phe Ile Lys Pro Leu Lys Ala
            100                 105                 110

Asn Gln Gln Phe Leu Val Tyr Cys Glu Ile Asp Gly Ser Gly Asn Gly
        115                 120                 125

Trp Thr Val Phe Gln Lys Arg Leu Asp Gly Ser Val Asp Phe Lys Lys
    130                 135                 140

Asn Trp Ile Gln Tyr Lys Glu Gly Phe Gly His Leu Ser Pro Thr Gly
145                 150                 155                 160

Thr Thr Glu Phe Trp Leu Gly Asn Glu Lys Ile His Leu Ile Ser Thr
                165                 170                 175

Gln Ser Ala Ile Pro Tyr Ala Leu Arg Val Glu Leu Glu Asp Trp Asn
            180                 185                 190

Gly Arg
```

<210> 6
<211> 650
<212> PRT
<213> Homo sapiens

<220>
<223> polypeptide of spot 182

<400> 6

```
Trp Cys Ala Val Ser Glu His Glu Ala Thr Lys Cys Gln Ser Phe Arg
1               5                   10                  15
```

```
Asp His Met Lys Ser Val Ile Pro Ser Asp Gly Pro Ser Val Ala Cys
            20                  25                  30

Val Lys Lys Ala Ser Tyr Leu Asp Cys Ile Arg Ala Ile Ala Ala Asn
        35                  40                  45

Glu Ala Asp Ala Val Thr Leu Asp Ala Gly Leu Val Tyr Asp Ala Tyr
    50                  55                  60

Leu Ala Pro Asn Asn Leu Lys Pro Val Val Ala Glu Phe Tyr Gly Ser
65                  70                  75                  80

Lys Glu Asp Pro Gln Thr Phe Tyr Tyr Ala Val Ala Val Val Lys Lys
                85                  90                  95

Asp Ser Gly Phe Gln Met Asn Gln Leu Arg Gly Lys Lys Ser Cys His
            100                 105                 110

Thr Gly Leu Gly Arg Ser Ala Gly Trp Asn Ile Pro Ile Gly Leu Leu
        115                 120                 125

Tyr Cys Asp Leu Pro Glu Pro Arg Lys Pro Leu Glu Lys Ala Val Ala
    130                 135                 140

Asn Phe Phe Ser Gly Ser Cys Ala Pro Cys Ala Asp Gly Thr Asp Phe
145                 150                 155                 160

Pro Gln Leu Cys Gln Leu Cys Pro Gly Cys Gly Cys Ser Thr Leu Asn
                165                 170                 175

Gln Tyr Phe Gly Tyr Ser Gly Ala Phe Lys Cys Leu Lys Asp Gly Ala
            180                 185                 190

Gly Asp Val Ala Phe Val Lys His Ser Thr Ile Phe Glu Asn Leu Ala
        195                 200                 205

Asn Lys Ala Asp Arg Asp Gln Tyr Glu Leu Leu Cys Leu Asp Asn Thr
    210                 215                 220

Arg Lys Pro Val Asp Glu Tyr Lys Asp Cys His Leu Ala Gln Val Pro
225                 230                 235                 240

Ser His Thr Val Val Ala Arg Ser Met Gly Gly Lys Glu Asp Leu Ile
                245                 250                 255

Trp Glu Leu Leu Asn Gln Ala Gln Glu His Phe Gly Lys Asp Lys Ser
            260                 265                 270
```

Lys Glu Phe Gln Leu Phe Ser Ser Pro His Gly Lys Asp Leu Leu Phe
275 280 285

Lys Asp Ser Ala His Gly Phe Leu Lys Val Pro Pro Arg Met Asp Ala
290 295 300

Lys Met Tyr Leu Gly Tyr Glu Tyr Val Thr Ala Ile Arg Asn Leu Arg
305 310 315 320

Glu Gly Thr Cys Pro Glu Ala Pro Thr Asp Glu Cys Lys Pro Val Lys
325 330 335

Trp Cys Ala Leu Ser His His Glu Arg Leu Lys Cys Asp Glu Trp Ser
340 345 350

Val Asn Ser Val Gly Lys Ile Glu Cys Val Ser Ala Glu Thr Thr Glu
355 360 365

Asp Cys Ile Ala Lys Ile Met Asn Gly Glu Ala Asp Ala Met Ser Leu
370 375 380

Asp Gly Gly Phe Val Tyr Ile Ala Gly Lys Cys Gly Leu Val Pro Val
385 390 395 400

Leu Ala Glu Asn Tyr Asn Lys Ser Asp Asn Cys Glu Asp Thr Pro Glu
405 410 415

Ala Gly Tyr Phe Ala Val Ala Val Val Lys Lys Ser Ala Ser Asp Leu
420 425 430

Thr Trp Asp Asn Leu Lys Gly Lys Lys Ser Cys His Thr Ala Val Gly
435 440 445

Arg Thr Ala Gly Trp Asn Ile Pro Met Gly Leu Leu Tyr Asn Lys Ile
450 455 460

Asn His Cys Arg Phe Asp Glu Phe Phe Ser Glu Gly Cys Ala Pro Gly
465 470 475 480

Ser Lys Lys Asp Ser Ser Leu Cys Lys Leu Cys Met Gly Ser Gly Leu
485 490 495

Asn Leu Cys Glu Pro Asn Asn Lys Glu Gly Tyr Tyr Gly Tyr Thr Gly
500 505 510

Ala Phe Arg Cys Leu Val Glu Lys Gly Asp Val Ala Phe Val Lys His
515 520 525

Gln Thr Val Pro Gln Asn Thr Gly Gly Lys Asn Pro Asp Pro Trp Ala

```
    530                 535                 540

Lys Asn Leu Asn Glu Lys Asp Tyr Glu Leu Leu Cys Leu Asp Gly Thr
545                 550                 555                 560

Arg Lys Pro Val Glu Glu Tyr Ala Asn Cys His Leu Ala Arg Ala Pro
                565                 570                 575

Asn His Ala Val Val Thr Arg Lys Asp Lys Glu Ala Cys Val His Lys
            580                 585                 590

Ile Leu Arg Gln Gln Gln His Leu Phe Gly Ser Asn Val Thr Asp Cys
        595                 600                 605

Ser Gly Asn Phe Cys Leu Phe Arg Ser Glu Thr Lys Asp Leu Leu Phe
    610                 615                 620

Arg Asp Asp Thr Val Cys Leu Ala Lys Leu His Asp Arg Asn Thr Tyr
625                 630                 635                 640

Glu Lys Tyr Leu Gly Glu Glu Tyr Val Lys
                645                 650


<210>  7
<211>  327
<212>  PRT
<213>  Homo sapiens

<220>
<223>  polypeptide of spot 183

<400>  7

Tyr Ala Ala Thr Ser Gln Val Leu Leu Pro Ser Lys Asp Val Met Gln
1               5                   10                  15

Gly Thr Asp Glu His Val Val Cys Lys Val Gln His Pro Asn Gly Asn
            20                  25                  30

Lys Glu Lys Asn Val Pro Leu Pro Val Ile Ala Glu Leu Pro Pro Lys
        35                  40                  45

Val Ser Val Phe Val Pro Pro Arg Asp Gly Phe Phe Gly Asn Pro Arg
    50                  55                  60

Lys Ser Lys Leu Ile Cys Gln Ala Thr Gly Phe Ser Pro Arg Gln Ile
65                  70                  75                  80

Gln Val Ser Trp Leu Arg Glu Gly Lys Gln Val Gly Ser Gly Val Thr
                85                  90                  95
```

```
Thr Asp Gln Val Gln Ala Glu Ala Lys Glu Ser Gly Pro Thr Thr Tyr
            100                 105                 110

Lys Val Thr Ser Thr Leu Thr Ile Lys Glu Ser Asp Trp Leu Ser Gln
        115                 120                 125

Ser Met Phe Thr Cys Arg Val Asp His Arg Gly Leu Thr Phe Gln Gln
    130                 135                 140

Asn Ala Ser Ser Met Cys Val Pro Asp Gln Asp Thr Ala Ile Arg Val
145                 150                 155                 160

Phe Ala Ile Pro Pro Ser Phe Ala Ser Ile Phe Leu Thr Lys Ser Thr
                165                 170                 175

Lys Leu Thr Cys Leu Val Thr Asp Leu Thr Thr Tyr Asp Ser Val Thr
            180                 185                 190

Ile Ser Trp Thr Arg Gln Asn Gly Glu Ala Val Lys Thr His Thr Asn
        195                 200                 205

Ile Ser Glu Ser His Pro Asn Ala Thr Phe Ser Ala Val Gly Glu Ala
    210                 215                 220

Ser Ile Cys Glu Asp Asp Trp Asn Ser Gly Glu Arg Phe Thr Cys Thr
225                 230                 235                 240

Val Thr His Thr Asp Leu Pro Ser Pro Leu Lys Gln Thr Ile Ser Arg
                245                 250                 255

Pro Lys Gly Val Ala Leu His Arg Pro Asp Val Tyr Leu Leu Pro Pro
            260                 265                 270

Ala Arg Glu Gln Leu Asn Leu Arg Glu Ser Ala Thr Ile Thr Cys Leu
        275                 280                 285

Val Thr Gly Phe Ser Pro Ala Asp Val Phe Val Gln Trp Met Gln Arg
    290                 295                 300

Gly Gln Pro Leu Ser Pro Glu Lys Tyr Val Thr Ser Ala Pro Met Pro
305                 310                 315                 320

Glu Pro Gln Ala Pro Gly Arg
                325
```

<210>  8
<211>  393
<212>  PRT
<213>  Homo sapiens

<220>
<223> polypeptide of spot 87

<400> 8

```
Glu Ile Gln Asn Ala Val Asn Gly Val Lys Gln Ile Lys Thr Leu Ile
1               5                   10                  15

Glu Lys Thr Asn Glu Glu Arg Lys Thr Leu Leu Ser Asn Leu Glu Glu
            20                  25                  30

Ala Lys Lys Lys Lys Glu Asp Ala Leu Asn Glu Thr Arg Glu Ser Glu
        35                  40                  45

Thr Lys Leu Lys Glu Leu Pro Gly Val Cys Asn Glu Thr Met Met Ala
    50                  55                  60

Leu Trp Glu Glu Cys Lys Pro Cys Leu Lys Gln Thr Cys Met Lys Phe
65                  70                  75                  80

Tyr Ala Arg Val Cys Arg Ser Gly Ser Gly Leu Val Gly Arg Gln Leu
                85                  90                  95

Glu Glu Phe Leu Asn Gln Ser Ser Pro Phe Tyr Phe Trp Met Asn Gly
            100                 105                 110

Asp Arg Ile Asp Ser Leu Leu Glu Asn Asp Arg Gln Gln Thr His Met
        115                 120                 125

Leu Asp Val Met Gln Asp His Phe Ser Arg Ala Ser Ser Ile Ile Asp
    130                 135                 140

Glu Leu Phe Gln Asp Arg Phe Phe Thr Arg Glu Pro Gln Asp Thr Tyr
145                 150                 155                 160

His Tyr Leu Pro Phe Ser Leu Pro His Arg Arg Pro His Phe Phe Phe
                165                 170                 175

Pro Lys Ser Arg Ile Val Arg Ser Leu Met Pro Phe Ser Pro Tyr Glu
            180                 185                 190

Pro Leu Asn Phe His Ala Met Phe Gln Pro Phe Leu Glu Met Ile His
        195                 200                 205

Glu Ala Gln Gln Ala Met Asp Ile His Phe His Ser Pro Ala Phe Gln
    210                 215                 220

His Pro Pro Thr Glu Phe Ile Arg Glu Gly Asp Asp Asp Arg Thr Val
225                 230                 235                 240
```

```
Cys Arg Glu Ile Arg His Asn Ser Thr Gly Cys Leu Arg Met Lys Asp
                245                 250                 255

Gln Cys Asp Lys Cys Arg Glu Ile Leu Ser Val Asp Cys Ser Thr Asn
            260                 265                 270

Asn Pro Ser Gln Ala Lys Leu Arg Arg Glu Leu Asp Glu Ser Leu Gln
        275                 280                 285

Val Ala Glu Arg Leu Thr Arg Lys Tyr Asn Glu Leu Leu Lys Ser Tyr
    290                 295                 300

Gln Trp Lys Met Leu Asn Thr Ser Ser Leu Leu Glu Gln Leu Asn Glu
305                 310                 315                 320

Gln Phe Asn Trp Val Ser Arg Leu Ala Asn Leu Thr Gln Gly Glu Asp
                325                 330                 335

Gln Tyr Tyr Leu Arg Val Thr Thr Val Ala Ser His Thr Ser Asp Ser
            340                 345                 350

Asp Val Pro Ser Gly Val Thr Glu Val Val Val Lys Leu Phe Asp Ser
        355                 360                 365

Asp Pro Ile Thr Val Thr Val Pro Val Glu Val Ser Arg Lys Asn Pro
    370                 375                 380

Lys Phe Met Glu Thr Val Ala Glu Lys
385                 390


<210>  9
<211>  319
<212>  PRT
<213>  Homo sapiens

<220>
<223>  polypeptide of spot 101

<400>  9

Glu Asn Glu Gly Phe Thr Val Thr Ala Glu Gly Lys Gly Gln Gly Thr
1               5                   10                  15

Leu Ser Val Val Thr Met Tyr His Ala Lys Ala Lys Asp Gln Leu Thr
            20                  25                  30

Cys Asn Lys Phe Asp Leu Lys Val Thr Ile Lys Pro Ala Pro Glu Thr
        35                  40                  45

Glu Lys Arg Pro Gln Asp Ala Lys Asn Thr Met Ile Leu Glu Ile Cys
    50                  55                  60
```

```
Thr Arg Tyr Arg Gly Asp Gln Asp Ala Thr Met Ser Ile Leu Asp Ile
65                  70                  75                  80

Ser Met Met Thr Gly Phe Ala Pro Asp Thr Asp Asp Leu Lys Gln Leu
                85                  90                  95

Ala Asn Gly Val Asp Arg Tyr Ile Ser Lys Tyr Glu Leu Asp Lys Ala
            100                 105                 110

Phe Ser Asp Arg Asn Thr Leu Ile Ile Tyr Leu Asp Lys Val Ser His
        115                 120                 125

Ser Glu Asp Asp Cys Leu Ala Phe Lys Val His Gln Tyr Phe Asn Val
    130                 135                 140

Glu Leu Ile Gln Pro Gly Ala Val Lys Val Tyr Ala Tyr Tyr Asn Leu
145                 150                 155                 160

Glu Glu Ser Cys Thr Arg Phe Tyr His Pro Glu Lys Glu Asp Gly Lys
                165                 170                 175

Leu Asn Lys Leu Cys Arg Asp Glu Leu Cys Arg Cys Ala Glu Glu Asn
            180                 185                 190

Cys Phe Ile Gln Lys Ser Asp Asp Lys Val Thr Leu Glu Glu Arg Leu
        195                 200                 205

Asp Lys Ala Cys Glu Pro Gly Val Asp Tyr Val Tyr Lys Thr Arg Leu
    210                 215                 220

Val Lys Val Gln Leu Ser Asn Asp Phe Asp Glu Tyr Ile Met Ala Ile
225                 230                 235                 240

Glu Gln Thr Ile Lys Ser Gly Ser Asp Glu Val Gln Val Gly Gln Gln
                245                 250                 255

Arg Thr Phe Ile Ser Pro Ile Lys Cys Arg Glu Ala Leu Lys Leu Glu
            260                 265                 270

Glu Lys Lys His Tyr Leu Met Trp Gly Leu Ser Ser Asp Phe Trp Gly
        275                 280                 285

Glu Lys Pro Asn Leu Ser Tyr Ile Ile Gly Lys Asp Thr Trp Val Glu
    290                 295                 300

His Trp Pro Glu Glu Asp Glu Cys Gln Asp Glu Glu Asn Gln Lys
305                 310                 315
```

<210> 10
<211>
<212> PRT
<213> Homo sapiens

<220>
<223> c3c polypeptide

<400> 10

Ser Pro Met Tyr Ser Ile Ile Thr Pro Asn Ile Leu Arg Leu Glu Ser
1 5 10 15

Glu Glu Thr Met Val Leu Glu Ala His Asp Ala Gln Gly Asp Val Pro
20 25 30

Val Thr Val Thr Val His Asp Phe Pro Gly Lys Lys Leu Val Leu Ser
35 40 45

Ser Glu Lys Thr Val Leu Thr Pro Ala Thr Asn His Met Gly Asn Val
50 55 60

Thr Phe Thr Ile Pro Ala Asn Arg Glu Phe Lys Ser Glu Lys Gly Arg
65 70 75 80

Asn Lys Phe Val Thr Val Gln Ala Thr Phe Gly Thr Gln Val Val Glu
85 90 95

Lys Val Val Leu Val Ser Leu Gln Ser Gly Tyr Leu Phe Ile Gln Thr
100 105 110

Asp Lys Thr Ile Tyr Thr Pro Gly Ser Thr Val Leu Tyr Arg Ile Phe
115 120 125

Thr Val Asn His Lys Leu Leu Pro Val Gly Arg Thr Val Met Val Asn
130 135 140

Ile Glu Asn Pro Glu Gly Ile Pro Val Lys Gln Asp Ser Leu Ser Ser
145 150 155 160

Gln Asn Gln Leu Gly Val Leu Pro Leu Ser Trp Asp Ile Pro Glu Leu
165 170 175

Val Asn Met Gly Gln Trp Lys Ile Arg Ala Tyr Tyr Glu Asn Ser Pro
180 185 190

Gln Gln Val Phe Ser Thr Glu Phe Glu Val Lys Glu Tyr Val Leu Pro
195 200 205

Ser Phe Glu Val Ile Val Glu Pro Thr Glu Lys Phe Tyr Tyr Ile Tyr
210 215 220

```
Asn Glu Lys Gly Leu Glu Val Thr Ile Thr Ala Arg Phe Leu Tyr Gly
225                 230                 235                 240

Lys Lys Val Glu Gly Thr Ala Phe Val Ile Phe Gly Ile Gln Asp Gly
                245                 250                 255

Glu Gln Arg Ile Ser Leu Pro Glu Ser Leu Lys Arg Ile Pro Ile Glu
            260                 265                 270

Asp Gly Ser Gly Glu Val Val Leu Ser Arg Lys Val Leu Leu Asp Gly
        275                 280                 285

Val Gln Asn Pro Arg Ala Glu Asp Leu Val Gly Lys Ser Leu Tyr Val
    290                 295                 300

Ser Ala Thr Val Ile Leu His Ser Gly Ser Asp Met Val Gln Ala Glu
305                 310                 315                 320

Arg Ser Gly Ile Pro Ile Val Thr Ser Pro Tyr Gln Ile His Phe Thr
                325                 330                 335

Lys Thr Pro Lys Tyr Phe Lys Pro Gly Met Pro Phe Asp Leu Met Val
            340                 345                 350

Phe Val Thr Asn Pro Asp Gly Ser Pro Ala Tyr Arg Val Pro Val Ala
        355                 360                 365

Val Gln Gly Glu Asp Thr Val Gln Ser Leu Thr Gln Gly Asp Gly Val
    370                 375                 380

Ala Lys Leu Ser Ile Asn Thr His Pro Ser Gln Lys Pro Leu Ser Ile
385                 390                 395                 400

Thr Val Arg Thr Lys Lys Gln Glu Leu Ser Glu Ala Glu Gln Ala Thr
                405                 410                 415

Arg Thr Met Gln Ala Leu Pro Tyr Ser Thr Val Gly Asn Ser Asn Asn
            420                 425                 430

Tyr Leu His Leu Ser Val Leu Arg Thr Glu Leu Arg Pro Gly Glu Thr
        435                 440                 445

Leu Asn Val Asn Phe Leu Leu Arg Met Asp Arg Ala His Glu Ala Lys
    450                 455                 460

Ile Arg Tyr Tyr Thr Tyr Leu Ile Met Asn Lys Gly Arg Leu Leu Lys
465                 470                 475                 480
```

```
Ala Gly Arg Gln Val Arg Glu Pro Gly Gln Asp Leu Val Val Leu Pro
                485                 490                 495

Leu Ser Ile Thr Thr Asp Phe Ile Pro Ser Phe Arg Leu Val Ala Tyr
            500                 505                 510

Tyr Thr Leu Ile Gly Ala Ser Gly Gln Arg Glu Val Val Ala Asp Ser
        515                 520                 525

Val Trp Val Asp Val Lys Asp Ser Cys Val Gly Ser Leu Val Val Lys
    530                 535                 540

Ser Gly Gln Ser Glu Asp Arg Gln Pro Val Pro Gly Gln Gln Met Thr
545                 550                 555                 560

Leu Lys Ile Glu Gly Asp His Gly Ala Arg Val Val Leu Val Ala Val
                565                 570                 575

Asp Lys Gly Val Phe Val Leu Asn Lys Lys Asn Lys Leu Thr Gln Ser
            580                 585                 590

Lys Ile Trp Asp Val Val Glu Lys Ala Asp Ile Gly Cys Thr Pro Gly
        595                 600                 605

Ser Gly Lys Asp Tyr Ala Gly Val Phe Ser Asp Ala Gly Leu Thr Phe
    610                 615                 620

Thr Ser Ser Ser Gly Gln Gln Thr Ala Gln Arg Ala Glu Leu Gln Cys
625                 630                 635                 640

Pro Gln Pro Ala Ala Ser Asn Leu Asp Glu Asp Ile Ile Ala Glu Glu
                645                 650                 655

Asn Ile Val Ser Arg Ser Glu Phe Pro Glu Ser Trp Leu Trp Asn Val
            660                 665                 670

Glu Asp Leu Lys Glu Pro Pro Lys Asn Gly Ile Ser Thr Lys Leu Met
        675                 680                 685

Asn Ile Phe Leu Lys Asp Ser Ile Thr Thr Trp Glu Ile Leu Ala Val
    690                 695                 700

Ser Met Ser Asp Lys Lys Gly Ile Cys Val Ala Asp Pro Phe Glu Val
705                 710                 715                 720

Thr Val Met Gln Asp Phe Phe Ile Asp Leu Arg Leu Pro Tyr Ser Val
                725                 730                 735

Val Arg Asn Glu Gln Val Glu Ile Arg Ala Val Leu Tyr Asn Tyr Arg
```

740 745 750

Gln Asn Gln Glu Leu Lys Val Arg Val Glu Leu Leu His Asn Pro Ala
755 760 765

Phe Cys Ser Leu Ala Thr Thr Lys Arg Arg His Gln Gln Thr Val Thr
770 775 780

Ile Pro Pro Lys Ser Ser Leu Ser Val Pro Tyr Val Ile Val Pro Leu
785 790 795 800

Lys Thr Gly Leu Gln Glu Val Glu Val Lys Ala Ala Val Tyr His His
805 810 815

Phe Ile Ser Asp Gly Val Arg Lys Ser Leu Lys Val Val Pro Glu Gly
820 825 830

Ile Arg Met Asn Lys Thr Val Ala Val Arg Thr Leu Asp Pro Glu Arg
835 840 845

Leu Gly Arg Ser Glu Glu Thr Lys Glu Asn Glu Gly Phe Thr Val Thr
850 855 860

Ala Glu Gly Lys Gly Gln Gly Thr Leu Ser Val Val Thr Met Tyr His
865 870 875 880

Ala Lys Ala Lys Asp Gln Leu Thr Cys Asn Lys Phe Asp Leu Lys Val
885 890 895

Thr Ile Lys Pro Ala Pro Glu Thr Glu Lys Arg Pro Gln Asp Ala Lys
900 905 910

Asn Thr Met Ile Leu Glu Ile Cys Thr Arg Tyr Arg Gly Asp Gln Asp
915 920 925

Ala Thr Met Ser Ile Leu Asp Ile Ser Met Met Thr Gly Phe Ala Pro
930 935 940

Asp Thr Asp Asp Leu Lys Gln Leu Ala Asn Gly Val Asp Arg Tyr Ile
945 950 955 960

Ser Lys Tyr Glu Leu Asp Lys Ala Phe Ser Asp Arg Asn Thr Leu Ile
965 970 975

Ile Tyr Leu Asp Lys Val Ser His Ser Glu Asp Asp Cys Leu Ala Phe
980 985 990

Lys Val His Gln Tyr Phe Asn Val Glu Leu Ile Gln Pro Gly Ala Val
995 1000 1005

```
Lys Val  Tyr Ala Tyr Tyr Asn  Leu Glu Glu Ser Cys  Thr Arg Phe
    1010                 1015                 1020

Tyr His  Pro Glu Lys Glu Asp  Gly Lys Leu Asn Lys  Leu Cys Arg
    1025                 1030                 1035

Asp Glu  Leu Cys Arg Cys Ala  Glu Glu Asn Cys Phe  Ile Gln Lys
    1040                 1045                 1050

Ser Asp  Asp Lys Val Thr Leu  Glu Glu Arg Leu Asp  Lys Ala Cys
    1055                 1060                 1065

Glu Pro  Gly Val Asp Tyr Val  Tyr Lys Thr Arg Leu  Val Lys Val
    1070                 1075                 1080

Gln Leu  Ser Asn Asp Phe Asp  Glu Tyr Ile Met Ala  Ile Glu Gln
    1085                 1090                 1095

Thr Ile  Lys Ser Gly Ser Asp  Glu Val Gln Val Gly  Gln Gln Arg
    1100                 1105                 1110

Thr Phe  Ile Ser Pro Ile Lys  Cys Arg Glu Ala Leu  Lys Leu Glu
    1115                 1120                 1125

Glu Lys  Lys His Tyr Leu Met  Trp Gly Leu Ser Ser  Asp Phe Trp
    1130                 1135                 1140

Gly Glu  Lys Pro Asn Leu Ser  Tyr Ile Ile Gly Lys  Asp Thr Trp
    1145                 1150                 1155

Val Glu  His Trp Pro Glu Glu  Asp Glu Cys Gln Asp  Glu Glu Asn
    1160                 1165                 1170

Gln Lys  Gln Cys Gln Asp Leu  Gly Ala Phe Thr Glu  Ser Met Val
    1175                 1180                 1185

Val Phe  Gly Cys Pro Asn
    1190
```

## Description

Technical field

The present invention relates to biomarkers for diagnosing and detecting the progression of neurodegenerative disorders, in particular of amyotrophic lateral sclerosis (also referred hereinafter as ALS).

State of the art

Amyotrophic Lateral Sclerosis (ALS), also known as Lou Gehrig's disease, is the most severe motor neuron disease: it is a devastating disorder of the central nervous system (CNS) with a multifactor aetiopathogenesis and a lethal course. ALS affects 5 in every 100,000 individuals each year [Julien, 2001], and is therefore the third most common cause of death in adults due to neurodegenerative diseases, after Alzheimer's disease (AD) and Parkinson's disease [source: Motor Neuron Disease Association].

There are both familial and sporadic forms of ALS. Familial ALS accounts for 5-10% of all cases and has been correlated to several genetic mutations: about 20% of familial cases are associated to mutations in the gene for superoxide dismutase 1 (SOD1), an ubiquitously expressed antioxidant protein. Familial ALS and sporadic ALS are clinically undistinguishable, suggesting a common pathogenesis, possibly triggered by heterogeneous molecular events [Bruijn et al., 2004].

From an anatomopathological point of view, ALS is characterized by a rapid and selective loss of motor neurons in the brain, brainstem and/or spinal cord, negatively affecting the strength, growth and function of muscles. Early symptoms of ALS are quite heterogeneous and may include arm, leg and trunk weakness, spasticity, breathing difficulty, mastication and deglutition difficulty, and slurred speech. Progressive atrophy of skeletal muscles and paralysis follow, the most common cause of lethality being respiratory failure generally within 3 to 5 years from the clinical onset [Belsh, 1996; Rowland, 1998].

There is currently no effective therapy for ALS. The only approved drug, riluzole (a compound with anti-glutamatergic activity), slightly prolongs survival and partially relieves symptoms, but has no effect on blocking the progression of neurodegeneration, regressing the symptoms and partially recovering motor functions [Lacomblez et al., 1996; Miller et al., 2002].

Biomedical research on ALS has, among its priorities, the discovery of biomarkers for the disease, as currently there are no clinical tools for the molecular diagnosis of ALS. As a matter of fact, although there are many ongoing studies, publications and patent applications on the subject, there are currently no ALS-specific biomarkers available for clinical use [Shaw e Williams, 2000; Bowser et al., 2006]. In particular, no biomarker has proven capable of discriminating ALS patients from individuals with non-neurological inflammatory processes, nor are there disease progression markers available. In the absence of biochemical parameters allowing to diagnose ALS at an early stage, diagnostics is based only on the clinical observation of the symptoms which are often serious and invalidating and presumably appear well after the related pathogenic molecular events have already occurred. This may be one of the reasons why the available treatments only have a limited effect. It should also be noted that, in the absence of effective therapies, an early diagnose would allow to immediately start a therapy and therefore prolong life expectancy of the patient [Lacomblez et al., 1996; Miller et al., 2002].

The absence of biomarkers also does not allow an efficient classification on molecular bases of the different phenotypes of ALS, a multifactorial and complex disease which probably includes different subtypes within its clinical definition [Shaw e Williams, 2000; Bowser et al., 2006].

The absence of specific biomarkers on the other side reflects the poor knowledge of the molecular mechanisms involved in the onset and development of ALS, with the consequence that a quantitative indication of the efficacy may not be drawn for compounds screened for therapy. The discovery of new biomarkers may therefore contribute to the understanding of the mechanisms of the disease, and thus to the development of possible new therapeutic targets.

Disclosure of the invention

In view of the above, the need for new specific ALS biomarkers results apparent. It is an object of the present invention to therefore provide polypeptides which are found in blood/plasma/serum and may be used as biomarkers, in particular for diagnosing and detecting the progression of ALS.

According to the present invention this object is achieved by means of polypeptides according to claim 1.

Definitions

Unless otherwise explicitly specified, the following terms have the following meaning.

In the present disclosure "identity percentage" and "% identity" between two sequences of amino acids (peptides) or nucleic acids (nucleotides) means the percentage of amino acid residues or identical nucleotides at corresponding positions in the two aligned sequences when ideally aligned.

To determine the "identity percentage" of two amino acid or nucleic acid sequences, the sequences are aligned with one another; to achieve an optimum comparison, gaps (i.e. deletions or insertions - which may possibly even be arranged at the ends of the sequences) may be introduced in the sequences. The amino acid and nucleotide residues at corresponding positions are therefore compared. When a position in the first sequence is occupied by the same amino acid or nucleotide residue that occupies the corresponding position in the second sequence, the molecules are identical in this position. The identity percentage between two sequences is a function of the number of identical positions shared by the sequences [i.e. % identity = (number of identical positions / total number of positions) X 100].

According to an advantageous embodiment, the sequences have the same length.

Advantageously, the compared sequences do not have any gaps (or insertions).

The identity percentage may be obtained by using mathematical algorithms. A non-limitative example of a mathematical algorithm used for the comparison of two sequences is the Karlin and Altschul algorithm [Proc. Natl. Acad. Sci. USA 87 (1990) 2264-2268] modified by Karlin and Altschul [Proc. Natl. Acad. Sci. USA 90 (1993) 5873-5877]. Such an algorithm is incorporated in the BLASTn and BLASTp software by Altschul [Altschul, et al, J. Mol. Biol. 215 (1990) 403-410].

In order to obtain alignments also in the presence of one or more gaps (or insertions) methods may be used which give a relatively high penalty to each gap (or insertion) and a lower penalty for each additional amino acid or nucleotide residue in the gap (such an additional amino acid or nucleotide residue is defined as an extension of the gap). High penalties will obviously determine optimum alignments with a lower number of gaps.

An example of a software adapted to carry out this kind of alignment is the BLAST software as disclosed in Altschul, et al., Nucleic Acids Res. 25 (1997) 3389-3402. For this purpose the BLASTn and BLASTp software may be used with default parameters. A BLOSUM62 matrix is usually employed with BLAST softwares.

An advantageous and non-limitative example of a software to carry out an optimum alignment is GCG Winsconsin Bestfit package (Winsconsin University, USA; Devereux et al., 1984, Nucleic Acids Research 12:387). Default parameters which provide a penalty of -12 for a gap and a penalty of -4 for each extension for an amino acid sequence are also used in this case.

In the present disclosure "homology percentage" and "% homology" between two amino acid or nucleic acid sequences means the percentage of homologous amino acid or nucleotide residues at corresponding positions in the two sequences when ideally aligned.

The homology percentage between two sequences is determined in substantially the same manner as described above for the determination of the identity percentage except that homologous positions and not only identical positions are considered in the computation.

As far as nucleotide sequences are concerned, two homologous positions have two nucleotides which are different but lead to the same amino acid.

As far as amino acid sequences are concerned, two homologous positions have two homologous amino acids, i.e. amino acids having similar chemical-physical properties, for instance amino acids belonging to the same groups such as: aromatic (Phe, Trp, Tyr), acid (Glu, Asp), polar (Gln, Asn), basic (Lys, Arg, His), aliphatic (Ala, Leu, Ile, Val), with a hydroxy-group (Ser, Thr), with a short side chain (Gly, Ala, Ser, Thr, Met). Substitutions among these homologous amino acids are not expected to change the phenotype of the proteins (conservative amino acid substitutions). Specific examples of conservative substitutions are known in this technical field and are disclosed in literature (for example, Bowie et al., Science, 247:1306-1310 (1990)).

Further examples of software and/or items related to the determination of alignments and of homology and/or identity percentages are indicated for example in US2008003202, US2007093443, WO06048777

In the present text, "corresponding position" means a position in an amino acid or nucleotide sequence corresponding (facing), upon alignment, to a determined position of a reference sequence.

Brief description of the figures

For a better understanding of the present invention, the invention is now also described with reference to the accompanying figures, in which:

- Figure 1A is a table listing the spots corresponding to the biomarkers for diagnosing ALS identified by means of two-dimensional electrophoresis (2DE), and the respective SEQ ID NO, Accession number, number of peptides of the Mascot identification, covered sequence, apparent isoelectrical point (pI), apparent molecular weight (MW), Mowse score of the Mascot identification (MS score);
- Figure 1B is a table listing the spot corresponding to the biomarker for detecting the progression of ALS identified by 2DE, and the respective SEQ ID NO, Accession number, number of peptides, covered sequence, apparent pI, apparent PM, MS score;

- Figure 2 represents a two-dimensional gel in which the spots listed in the table of Figure 1A are highlighted;
- Figure 3 is a table showing, for each of the spots identified by 2DE, the average values of the percentage volumes (%Vol) of each group of individual tested plasma samples (i.e. group of ALS patients, group of cardiovascular patients (INF) and group of healthy controls (CTR)), obtained as disclosed in the following, the standard deviations corresponding to the average %Vol within the group, and the values of statistic p expressing the significance of each spot in distinguishing the ALS group with respect to each of two reference groups on the basis of the %Vol;
- Figure 4A is a diagrammatic representation of the three-dimensional magnification of spot 8 detected with Coomassie staining, as disclosed in the following;
- Figure 4B is an image of spot 8 on pools of plasma samples from healthy controls (A) and ALS patients (B). In the top panels, spot 8 (indicated by an arrow) is shown by Coomassie staining, while in the bottom panels it is shown by 2DE Western blotting;
- Figure 5 is a graph showing the levels of the biomarker corresponding to spot 8 in individuals analysed in the form of %Vol;
- Figure 6 is a histogram of the values of the diagnostic function DF, computed as disclosed in the following, for each of the individuals included in the study;
- Figure 7A is a graph which shows the trend of the %Vol of spot 101 in 10 ALS patients subjected to riluzole therapy, comparing two samples taken at an interval of 10-12 months for each patient;
- Figure 7B is a graph which shows the trend of the %Vol of spot 101 in 8 ALS patients subjected to riluzole + lithium therapy, comparing two samples taken at an interval of 3-5 months for each patient;
- Figure 7C is an image of spot 101 shown by Western Blotting on 2 pools of plasma samples - Time 1 and Time 2 - taken at an interval of 10-12 months.
- Figure 8 shows the minimum amino acid sequences of the albumin protein fragments corresponding to spot 65 (SEQ ID NO: 3, 110 (SEQ ID NO: 2), 8 (SEQ ID NO: 1), 66 (SEQ ID NO: 1), 34 (SEQ ID NO: 4), aligned with respect to the amino acid sequence of complete albumin (Accession number: 28592); and
- Figure 9 contains the image of a two-dimensional gel showing spot 101 (listed in the table of Figure 1B).

## Detailed description of the invention

The identification of novel biomarkers was carried out by screening plasma proteins. The passage of molecules from the CNS to plasma allows the direct identification in the plasma of biomarkers coming from disease sites, which are added to the possible alteration of specific plasma markers. Furthermore, the sampling of peripheral fluids allows for the collection of a greater number of samples during the discovery step of the biomarkers, providing a greater statistical robustness of the results obtained.

A two-dimensional electrophoresis (2DE) screening of the plasma proteome of patients suffering from ALS was carried out comparing the latter with healthy individuals and non-neurological patients sampled shortly after a heart-stroke or coronary-stroke (in the following referred to as cardiovascular patients).

The concept at the basis of applied proteome research is that most diseases result in a variation in the amount of proteins and peptides in the body fluids and tissues. Proteomic strategies may reveal the disruption of the balance in the distribution of different protein isoforms or modifications in proteins such as those deriving from oxidative stress, directly or indirectly related to the aetiological mechanisms [Perluigi et al., 2005]: For this reason much of the current applied biochemical research is directed to the study of the proteome of biological fluids to identify biomarkers related to different pathological conditions. As regards complex biological mixtures such as blood, different proteomic analyses have already identified proteins specifically associated to non-genetic diseases and unrelated to tumours [Aivado et al., 2007; Kim et al., 2007; Li et al., 2007; Avasarala et al., 2005].

It should be noted that not always are the identified biomarkers rare proteins or fragments thereof, or proteins or parts of proteins the expression of which is peculiar and rare. On the contrary, many publications report disease biomarkers which consist of modified forms of common and ubiquitary proteins such as albumin and proteins related thereto [Yagame et al., 1995; Kaiser et al., 2004; Funding et al., 2005; German et al., 2007]. An important advantage of proteomics based on 2DE consists in the possibility of identifying alterations in the amount of fragments and modified forms of proteins for which the level of the intact molecule does not significantly change [Finehout et al., 2007]. Peculiar protein fragments specific for ALS and other diseases with a strong apoptotic component, may derive from the selective activation of proteases and specific degradation pathways, which are usually active at low levels [Ilzecka et al., 2001]. In particular, as far as neurodegenerative diseases are concerned, it has been shown that C-terminal fragments of albumin present in the cerebrospinal fluid (CSF) of patients with AD are specific disease biomarkers, probably because the disease implies alterations in the degradation process of albumin [Finehout et al., 2007]. It has also been shown that albumin fragments have toxic activity on organotypic cultures of cholinergic neurons and on primary cultures of astrocytes [Moser and Humpel, 2007]. Therefore, peptides deriving from the degradation of albumin or other abundant plasma proteins could represent not only an epiphenomenon of the disease, but also of the main players in the pathogenesis.

For the proteomic study that has led to the identification of the biomarkers of the disease, the plasma proteome of three groups of individuals was considered:

- Healthy subjects (controls, n = 14)
- Cardiovascular patients, within 6 hours of heart-stroke or coronary-stroke (n = 8)
- ALS patients (n = 27)

The cardiovascular patients were included in the study as a "filter" to eliminate the biomarkers that discriminate ALS patients from controls only due to the inflammatory process ongoing in the patients. The plasma proteome of two groups of ALS patients was considered for the proteomic study that led to the identification of the progression biomarker: A study was first carried out on 10 patients subjected to a treatment with riluzole (analysing two samples - T1 and T2 - for each patient performed at an interval of 10-12 months). The result was then confirmed by a second study on 8 patients subjected to a treatment with riluzole + lithium (analysing two samples - T0-lithium and T1-lithium - for each patient performed at an interval of 3-5 months). Fresh blood from all patients was sampled by standard methods with their informed consent.

The plasma samples were then centrifuged at 400 g for 10' at 4°C. Albumin was not depleted from the sample, not only because albumin is a carrier protein which may bind to interesting markers, but also because (as previously disclosed) modified forms of albumin differentially present in the disease at issue may have a diagnostic role. The depletion of albumin may therefore lead to loss of useful biomarkers [Kawakami et al., 2005; German et al., 2007].

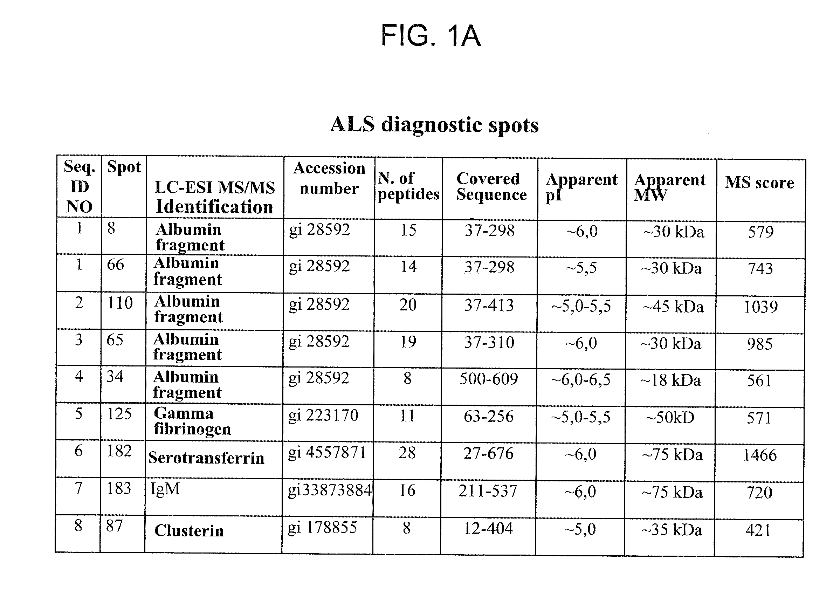

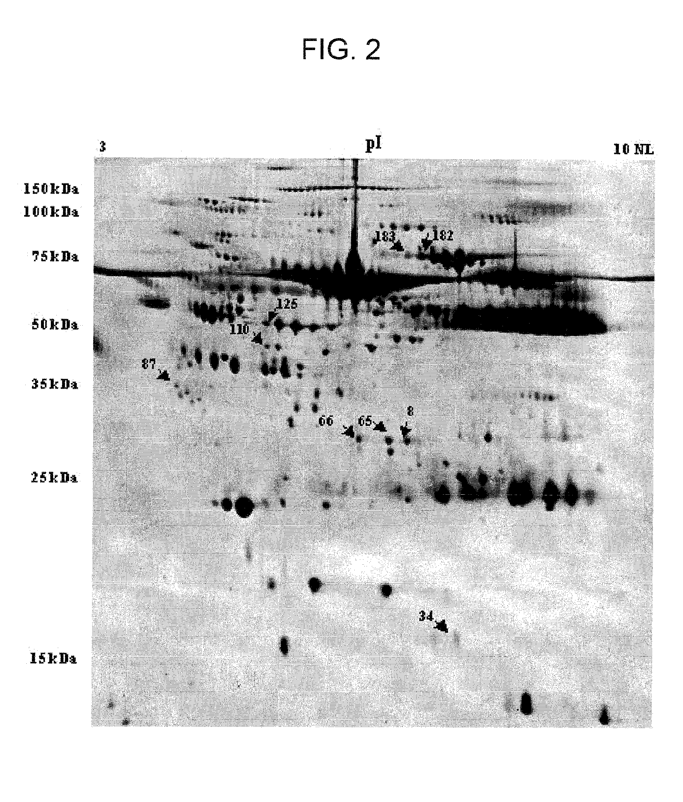

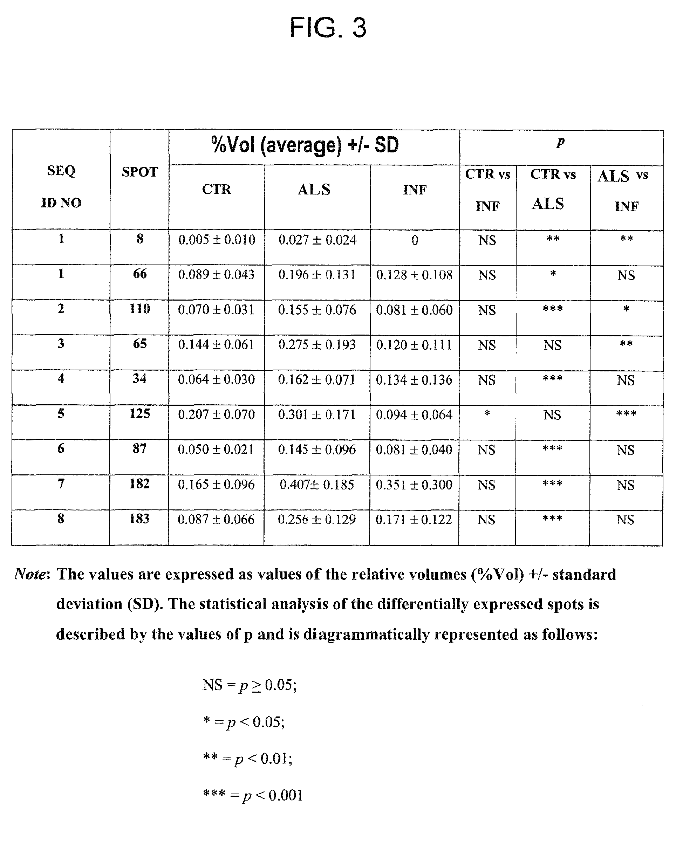

Once the samples were prepared, 2DE was performed for each sample and for at least one technical replicate according to Jacobs et al. [2001] with some modifications. In brief, 6 μl (about 400 μg of total protein in case of Coomassie staining), or 1,5 μl (about 100 μg of total protein in case of Sypro staining) of each plasma sample were heated for 5' at 95°C with 10 μl of SDS 5% w/v, DTT 2.5% w/v, and diluted to 330 μl with 7 M urea, 2 M thiourea, 4% w/v CHAPS, 0.5% v/v of IPG buffer 3-10 NL (GE Healthcare, Uppsala, Sweden), and traces of bromophenol blue, as in Hughes et al., 1992. The sample was then loaded on 18 cm IPG 3-10 NL strips (GE Healthcare, Uppsala, Sweden) for isoelectrofocusing (IEF) by in-gel rehydration (2 h at 0 V, 12 h at 30 V). IEF was then performed at 20°C on an IPGphor apparatus (GE Healthcare) as follows: 500 V at 500 V/hr, 1,000 V at 1,000 V/hr with a linear gradient; 8,000 V at 13,500 V/hr with a linear gradient, 8,000 V at 72,000 V/hr. Prior to SDS-PAGE (SDS-polyacrylamide gel denaturing electrophoresis), the IPG strips were equilibrated twice for 15' in a buffer containing 50 mM Tris-HCl pH 8.8, 6 M urea, 30% v/v glycerol, 2% w/v SDS, and traces of bromophenol blue, containing 1% w/v DTT for the first equilibration step and 2.5% w/v iodoacetamide for the second one. SDS-PAGE was
performed on 12.5% polyacrylamide gels (1.5 mm thick) according to Laemmli [1970] but without stacking gel, using a Hoefer SE600 apparatus (GE Healthcare). The second dimension was run at 60 mA/gel at 16°C until the bromophenol blue dye front reached the bottom of the gel. Proteins with a molecular weight (MW) in the range between 15 and 100 kDa and with an isoelectric point (pI) in the range between 4.5 and 8.5 were used as standards for the calibration of the MWs and of the pIs. The gels were coloured with Coomassie brilliant blue R350 (Sigma, San Diego, US) or with Sypro Ruby (Molecular Probes, Invitrogen). Stained gels were scanned with an ImageMaster Labscan V3.0 scanner (GE Healthcare) for the gels stained with Coomassie blue or with a CCD camera (Perkin Elmer) for the gels coloured with Sypro. Images were analysed with the ImageMaster 2-DE Platinum 5.0 software (GE Healthcare). For each group, a reference gel was chosen, i.e. the gel containing the highest amount of well focalized spots. Spots were detected and gels in turn matched with their respective reference. Spots present in more than 70% of the gels in a given group were used to create a synthetic gel (average gel) that represents the average proteome of each group. The three average gels were subsequently matched to detect the spots shared by the three examined populations; the subsequent quantitative analysis consisted in comparing the corresponding percentage volumes (%Vol) of each of these spots in the three populations. For each spot, the %Vol was calculated as an integral of the volume of each spot stained with Coomassie blue (area of the spot multiplied by its intensity) normalised by the sum of the volumes of all of the spots in the reference average gel. Spots, which consistently and significantly varied among the three populations, were extracted through a non-parametric ANOVA (Kruskal-Wallis test), plus a multiple comparisons post-test (Dunn's test), and p values $< 0.05$ were considered statistically significant. The spots identified in this manner are listed in Figure 1A and shown in Figure 2 (spot 65, 66, 110, 125, 182, 183, 87, 34, i.e. SEQ ID NO: 1-8, excluding spot 8). The average %Vol values and the standard deviations for each of the spots are summarised in Figure 3, highlighting the differences among the ALS patients and the rest of the individuals considered. Spot 110 allows to distinguish ALS patients both from controls and from cardiovascular patients. The experimental variability among technical replicates of the same sample has been determined by comparing the different %Vol obtained, and did not result in any case above 0.8 times (average variability: 0.45 times; minimum variability: 0.2 times).

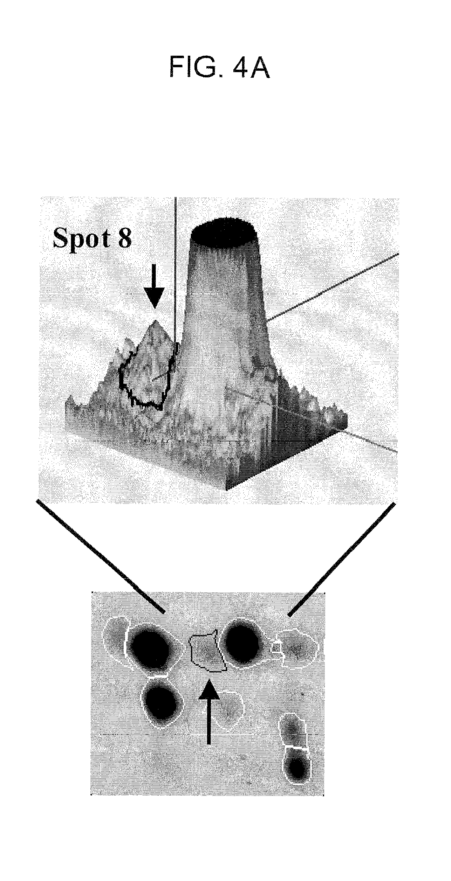

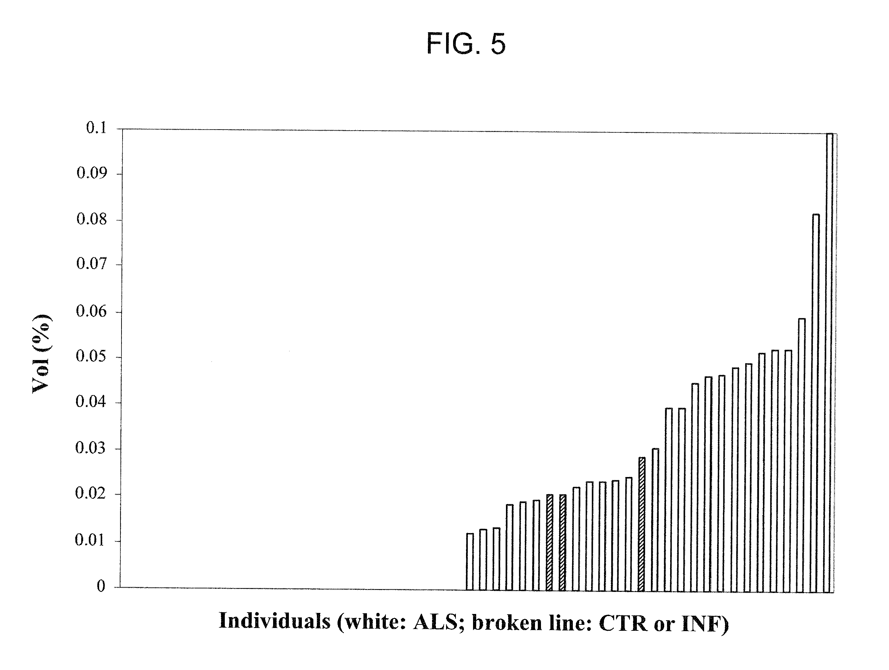

After the quantitative analysis was completed in the manner disclosed above, unmatched spots were subjected to a qualitative analysis, in order to identify those proteins or protein fragments representative of the ALS patient group only, and absent in the other groups, or vice-versa (qualitative analysis). Spot 8 (SEQ ID NO: 1) is also an identified ALS marker (Figure 1A, Figure 2; the three-dimensional magnification of spot 8 stained with Coomassie blue appears in Figure 4A; the levels of this marker in the tested individuals are shown in Figure 5).

Considering all of the 9 spots, a post-test discriminating analysis has been performed to assay the ability thereof to classify each individual in the appropriate population, on the basis of the measured %Vol (software: StatistiXL). The overall correct prediction efficiency reaches 89.8% with the correct identification of all of the ALS patients and of 81.5% of ALS patients. The same analysis has led to the formulation of the following linear function (Diagnostic Function, DF):

$$DF = - 3.349\ \%Vol(182) + 8.688\ \%Vol(183) - 1.146\ \%Vol(65) + 5.536\ \%Vol(110) + 1.652\ \%Vol(87) + 2.630\ \%Vol(125) + 3.026\ \%Vol(34) - 2.426\ \%Vol(66) + 30.098\ \%Vol(8) - 2.38$$

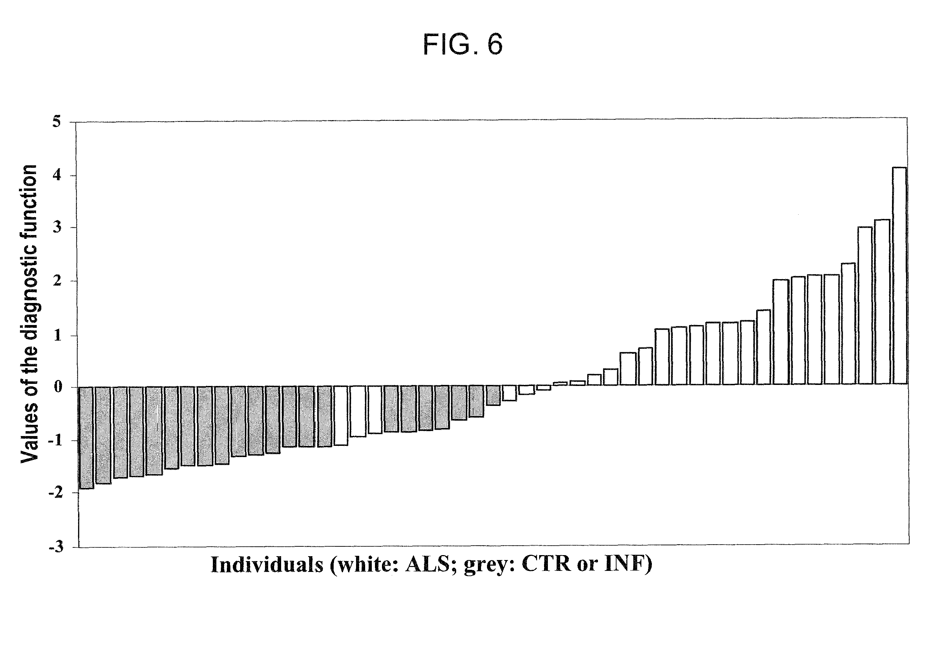

in which %Vol(n) indicates the %Vol of spot n, according to the numbering indicated on the 2DE map of Figure 2. The correspondence between the numbering of the spots considered for the computation of the DF as shown in Figure 2 and of their respective SEQ ID NOs, used elsewhere in the present patent, has been shown in Figure 1A. The value of the DF for each of the subjects included in the study is shown in Figure 6. A positive value of this function is clearly a diagnostic parameter for ALS, while a negative value tends to be associated to individuals not suffering from ALS.

The average values of the DF, the corresponding standard deviations and the size of the considered groups have been taken as input values for the following Power Analysis, comprising the computation of Cohen's D parameter, which accounts for the effect of the size of the sample in the determination of the normalised size effect, as disclosed by Cohen [1992] and by Hedges and Olkin [1985]. The Power Analysis which was performed therefore provides a measure of the confidence by which the same size effect may be observed on the entire population.

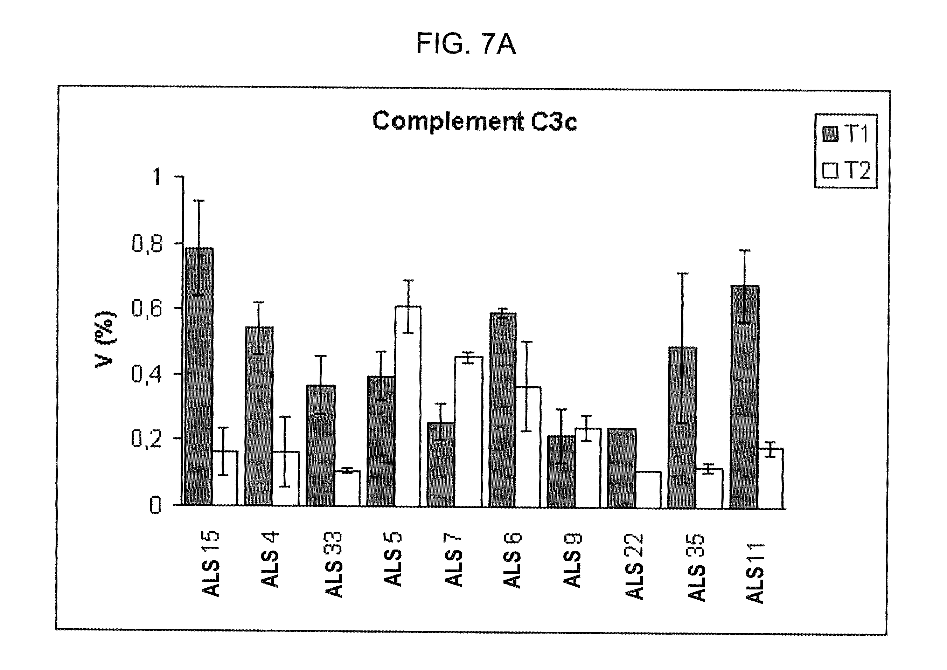

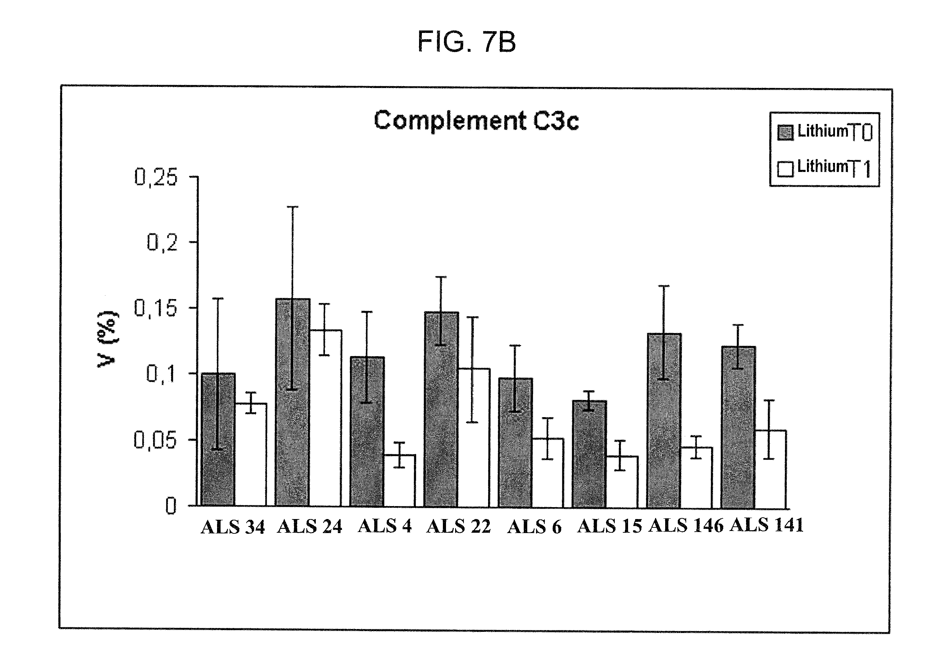

As regards the disease progression biomarkers, a non-parametric analysis (Mann-Whitney test) was carried out with a threshold of $p \leq 0,05$ in order to identify the spots which vary consistently and significantly between the two populations of samples (T1 vs T2, T0-lithium vs T1-lithium), as shown in Figures 7A and 7B: Spot 101 is an ALS progression biomarker.

Once the biomarkers were identified in the disclosed manner, the identity thereof was determined by mass spectrometry. In particular, the selected protein spots were excised manually from the gels and destained overnight with 40% ethanol in 25 mM ammonium bicarbonate, washed twice with 25 mM ammonium bicarbonate, three times with acetonitrile, and dried. Each gel fragment was rehydrated in 25 mM ammonium bicarbonate containing 0.6 μg of modified porcine trypsin and digested overnight at 37°C. Peptides were extracted by sonication in 25 mM ammonium bicarbonate, loaded onto a ZORBAX 300 SB C18 RP column (75 μm x 150 mm, 3.5 μm particles, Agilent, Santa Clara, CA, USA) and eluted with a gradient of acetonitrile from 5% to 80% (containing 0.1% formic acid) at a flow rate of 0.3 μl/min by an HP 1100 nanoLC system coupled to a XCT-Plus nanospray-ion trap mass spectrometer (Agilent) (elsewhere referred to as LC-ESI MS/MS. MS parameters were the following: scan range m/z = 100-2,200, scan speed 8,100 m/z s-1, dry gas flow 5 1/min, dry temperature 300°C, capillary 1.8 kV, skimmer 40 V, ion charge control (ICC) target 125,000, maximum accumulation time 300 ms. Positively charged peptides were automatically isolated and fragmented, and spectra were deconvoluted by the DataAnalysis software version 3.4 (Bruker Daltonics, Bremen, Germany). Mass spectrometry data obtained by LC-ESI MS/MS were fed to the Mascot search algorithm for searching against the NCBI non-redundant database (http://www.matrixscience.com - mass tolerance for the monoisotopic peak masses was set to 1.8 Da for the parent ion or 0.8 Da for the fragments; the maximum number of non-cut sites per peptide was 3). Allowed modifications were cysteine carbamidomethylation and methionine oxidation. Hits with a probability-based Mowse score higher than 47 were considered significant ($p < 0.05$).

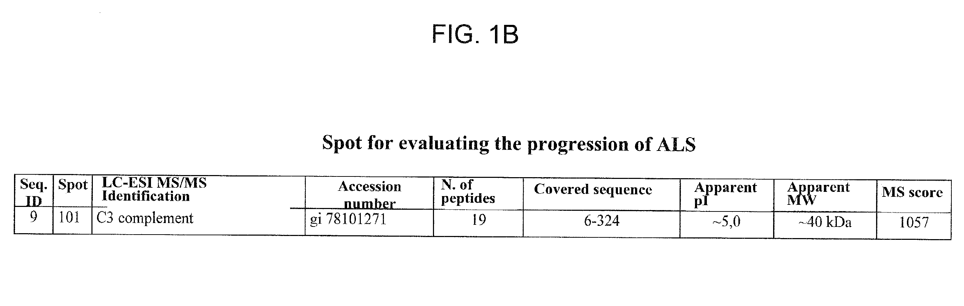

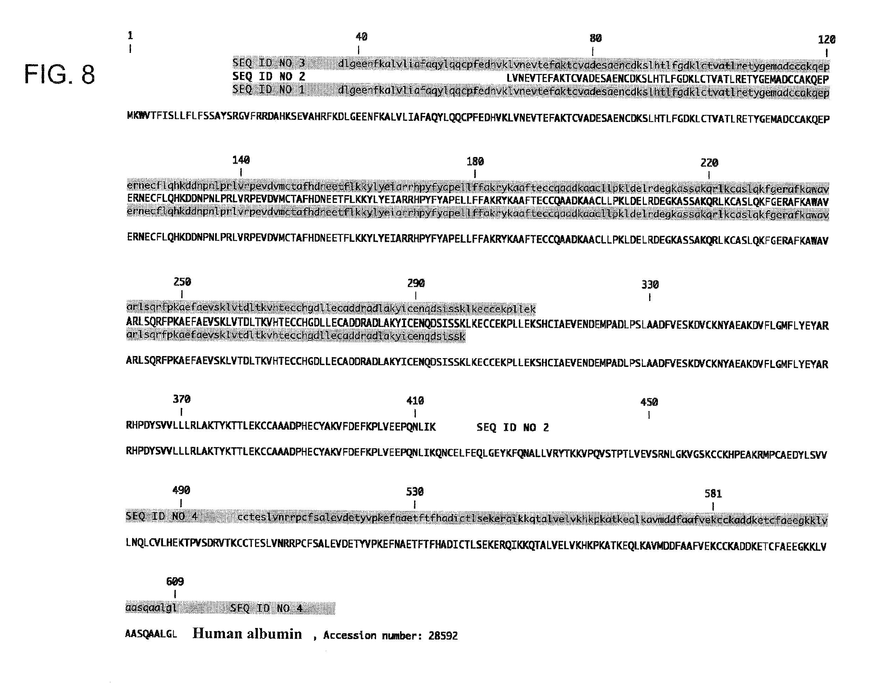

The protein identity of the biomarkers obtained in this manner is shown in Figure 1 and indicated with the corresponding SEQ ID NOs. As clearly results, albumin fragments (SEQ ID NO:1-4) are predominant. 1-4). The minimum amino acid sequence of these fragments is shown in Figure 1A and diagrammatically shown in Figure 8 as compared to the whole albumin sequence, identified by Accession Number 28592, which results being the reference sequence for human serum albumin. Fragments of this sequence have been identified in the present study as the biomarkers which are the object of the present patent.

"Minimum fragment sequence" means the sequence obtained by examining the peptides deriving from the tryptic digestion of a fragment, by sorting the peptides on the basis of the sequence of the native protein, and obtaining the sequence included between the N-terminal amino acid of the first peptide and the C-terminal amino acid thereof. This sequence is included within the fragment at issue, but is not limited thereto. Further amino acids may be present both at the N-terminal and at the C-terminal included between the identified tryptic sites and the following tryptic site (in an N or C-terminal direction). Therefore, although the fragments corresponding to spot 66 and spot 8 in Figure 1A (highlighted in the two-dimensional map of Figure 2) have the same minimum sequence (SEQ ID NO: 1), they are probably different at the C-terminal (as both minimum sequences start at the N-terminal end of mature albumin). In particular, as there are many acidic residues immediately downstream of the minimum sequence, it is possible that the fragment corresponding to spot 66 has some C-terminal acidic residues more than the fragment corresponding to spot 8, in accordance with its more acidic isoelectrical point (Figure 2, Figure 8).

The fragmentation of albumin in vivo is altered in many conditions, among which the after-effects of hematopoietic stem cell transplant [Kaiser et al., 2004], exocrine pancreatic damage [Walgren et al., 2007], acute corneal rejection [Funding et al., 2005], meningococcal sepsis [Holland et al., 2001], diabetic nephropathy [Yagame et al., 1995], ischemic heart disease, acute inflammation, endotoxicosis and ageing [Bito et al., 2005]. As regards the CNS and neurodegenerative diseases, specific albumin fragments have already been reported as being biomarkers: for instance, some C-terminal fragments of albumin present in the CSF represent specific biomarkers for AD [Finehout et al., 2007]. Furthermore, a specific serum albumin fragment has been found to be increased 2.8 times in a mouse model of muscle dystrophy [Doran et al., 2006]. As ALS is characterised by an extensive activation of serum proteases [Ilzecka et al., 2001; Demestre et al., 2006] and by a strong oxidative stress [Barber et al., 2006], it is feasible that some degradation mechanisms typical of the disease produce specific albumin fragments, such as those included in the set of biomarkers disclosed in Figure 1A and shown in Figure 8 against the sequence of the whole albumin.

Another biomarker which was identified and corresponds to spot 182 (Figure 1A and Figure 2; SEQ ID NO: 6), is a glycoform of transferrin. It should be recalled that, in ALS patients, transferrin accumulates in Bunina bodies [Mizuno et al., 2006], that the SOD1 protein modulates the expression of the transferrin receptor [Danzeisen et al., 2006], and finally that defects in the expression of alsin cause the intracellular accumulation of transferrin in motoneuron cultures [Jacquier et al., 2006]. If singularly considered, and not in combination with the other biomarkers disclosed in the present invention, transferrin would have a limited value as an ALS biomarker, as transferrin glycoforms are involved in an aspecific manner in different neurodegenerative and non-neurodegenerative diseases [Zeman et al., 2000; Brettschneider et al., 2008]. However, we have verified that the inclusion of these two spots in the identified set of biomarkers increases the overall diagnostic power of the set, probably because, as recalled, there are some mechanisms which lead to the alteration of transferrin in ALS patients.

Another biomarker that was identified and corresponds to spot 183 (SEQ ID NO: 7) is the constant chain of IgMs. The involvement of IgMs in ALS is well documented and mainly based on serological evidence on patients. High anti-GM1 ganglioside IgM titres are commonly found in patients with peripheral neuropathies and neuromotory syndromes [Pestronk, 1991]. More recently, high titres of IgMs against GM2 and GD2 were also dosed [Mizutani et al., 2003]. Noteworthy, IgM is the isotype of serum immune responses reported against neurofilament proteins [Couratier et al., 1998]. Therefore, in general, the IgM fragment identified as an ALS marker could derive from the IgM related immune response, reported in more than one study on ALS patients.

Another biomarker identified with the disclosed method is chain A of gamma-fibrinogen (spot 125, SEQ ID NO: 5). Although fibrinogen is not synthesised in the CNS, the increase of fibrinogen gamma A chain in the CSF is thought to be connected to blood-CSF barrier damage and fibrinogen is generally regarded as a marker of inflammation associated to neurological diseases, since it is known that the nervous system is especially able to produce fibrin receptors and fibrin-dependent intracellular signalling molecules under inflammatory conditions [discussed in Akassoglou e Strickland, 2002]. Recent studies have shown that macrophages in the CNS and Schwann cells in the peripheral nervous system are the two cytotypes most commonly involved in phenomena correlated with extravasation of fibrinogen and products resulting from the degradation thereof. Impairment of the fibrinolysis pathway is closely associated to the pathogenesis of MS, for which neuroinflammation is one of the main pathological features [Adams et al., 2004]. Furthermore, in the CSF of AD patients, an increase in chain A of gamma-fibrinogen has a role as disease biomarker, although this increase may be merely due to damage of the hematoencephalic barrier [Lee et al., 2007].

The last ALS biomarker that was identified (spot 87; SEQ ID NO: 8) was found to be a form of clusterin (Apo J). An increase in the mRNA for clusterin was shown by in situ hybridisation in areas of active neurodegeneration of the spinal cord [Grewal et al., 1999]. Clusterin may have a complex role in neurodegenerative processes: as well as having an inhibiting activity on the cell membrane anchoring complex, this multifunctional glycoprotein may promote cell aggregation and serve as molecular chaperone, preventing the aggregation of denatured proteins. The increase of the mRNA level of clusterin and of the protein itself may be detected in cerebral ischemic damage and in many neurological diseases, among which AD, multiple sclerosis and epilepsy. In some cells, the induction of the expression of clusterin is associated to apoptosis; non-neural cells engineered so as to produce reduced amounts of clusterin are more sensitive to oxidative stress [Grewal et al., 1999].

Among these 9 biomarkers, spot 8 has proved especially efficient:

- it allows to discriminate the population of ALS patients from both control populations (healthy and cardiovascular patients);

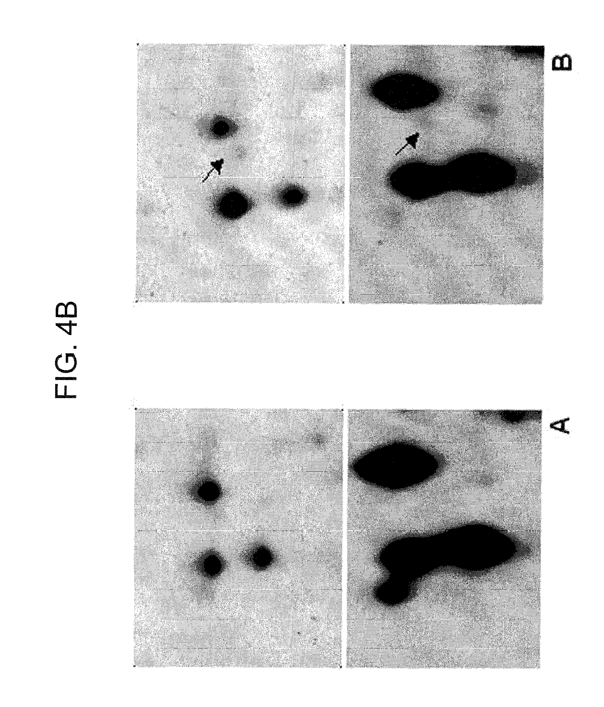

- the different expression of spot 8 in ALS patients with respect to healthy controls has also been tested by 2DE Western blotting (Figure 4B).

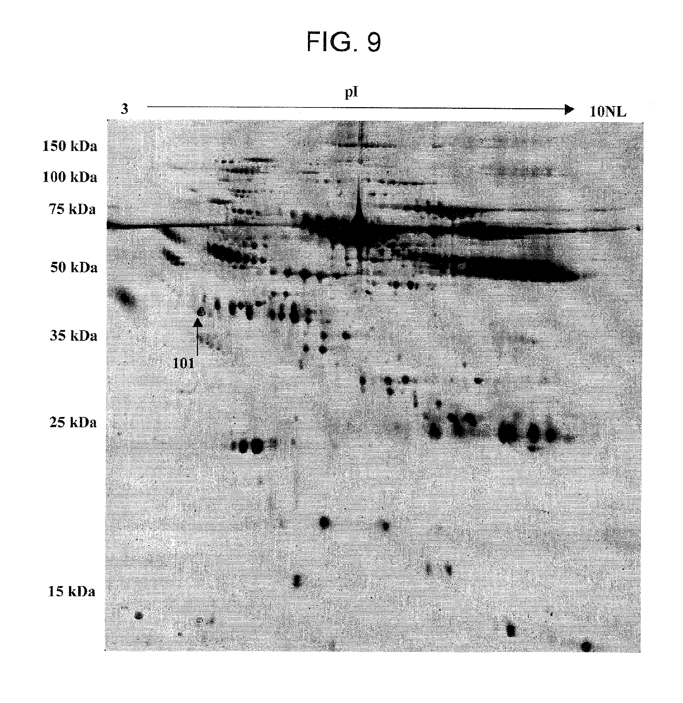

As regards the ALS progression biomarker, the analysis by LC-ESI MS/MS has identified it as complement component 3, in particular fragment 2 of chain α' of C3c (SEQ ID NO: 9). In non-denaturing conditions, this fragment is bound by disulphide bonds to other 2 polypeptide chains again deriving from complement C3: chain β' and fragment 2 of chain α' (polypeptide c3c SEQ ID NO: 10) (http://www.uniprot.org/). C3c fragments have already been identified by 2DE as peripheral ALS markers [Goldknopf et al., 2006]: however the fragments reported in Goldknopf et al. do not correspond to the specific fragment disclosed in the present invention, as is apparent from the totally different pI (and therefore from the different position in the 2DE maps obtained from serum or plasma, an example of which is shown in Figure 9). Furthermore, C3c has never been involved in the progression of the disease, and therefore the C3c fragment corresponding to spot 101 represents a truly new ALS progression biomarker.

It should be clear at this point to the person skilled in the art that any combination of disclosed biomarkers, with different statistical power, may be used for the differential diagnosis of ALS with respect to other neurodegenerative diseases, as well as for its progression. Furthermore, each combination of such biomarkers may be used together with other biomarkers to obtain a better predictive and statistical power. For example, the disclosed biomarkers, and in particular the %Vol evaluated by 2DE, may be used in combination with the ALS serum markers discovered by Goldknopf et al. [2006], both for the diagnosis and for the evaluation of the stage of progression of the disease. It is apparent that such combinations fall within the aim and scope of the present patent, as do other combinations of other types of biomarkers and/or physiological and/or diagnostic markers.

<u>Description of one or more embodiments</u>

The diagnostic procedure object of the present invention may be carried out by different embodiments.

By way of mere example, the following paragraph discloses an embodiment based on the sampling of blood from subjects to be tested, on the quantification by 2DE of the identified biomarkers, on the computation of a diagnostic function to identify the presence of ALS and on the evaluation of the stage of progression of the disease by comparing the amount of C3c on 2DE between diachronic samples.

As disclosed in the paragraph directed to the variants of the suggested method, it should be understood that the procedure disclosed in detail in the following is one among the many possible procedures which exploit the same set of biomarkers, which procedures must be considered, as a whole, as falling within the spirit and the scope of the present patent.

In particular, the present invention is based on the discovery of a set of ALS biomarkers, the amount of which is correlated with the presence of the disease (all of the biomarkers indicated in Figure 1A, i.e. all except C3c) or with its progression (only C3c, Figure 1B). The amount of these biomarkers may be evaluated by 2DE, as previously disclosed, but it is apparent to a person skilled in the art that any other evaluation method of the level of one or more of the biomarkers shown in Figure 1 falls within the scope and spirit of the present patent application. By mere way of example, the biomarkers may be quantified by one or more of the following alternative techniques:

1. Western blot
2. Enzyme-Linked ImmunoadSorbent Assay (ELISA)
3. High Pressure Liquid Chromatography (HPLC)
4. Mass spectrometry

Furthermore, a variant falling within the scope of the present patent application consists in using any numerical combination of the amount of some or all the disclosed biomarkers to compute a different diagnostic (linear or non-linear) function or derive any statistical parameter so as to obtain a score useful for the diagnosis of ALS or for the evaluation of its progression.

It should also be understood that any combination of the present biomarkers with other diagnostic methods for ALS or other neurological disorders must be considered to be part of the present patent application.

It should finally be understood that, although the use of human blood samples is preferable as compared to other biological material, the testing and use of any combination of biomarkers shown in Figure 1 in biological samples other than human blood must be part of the present patent application.

<u>Diagnostic procedure</u>

In order to diagnose ALS in an individual, or evaluate the stage of progression of the disease, the following paragraphs disclose:

(1) a method for quantifying the biomarkers object of the present patent;
(2) a method for diagnosing ALS based on the quantification of previous item (1);
(3) a method for evaluating the stage of progression of ALS based on the quantification of the C3c biomarker obtained with the procedure of item (1).

(1). QUANTIFICATION OF THE BIOMARKERS

The plasma of the individuals involved was obtained by standard methods. Once the plasma samples were prepared (by centrifugation at 4°C for 10' at 400 g), a 2DE experiment was carried out for each sample and for the corresponding technical replicate, according to Jacobs et al. [2001] with some modifications. In brief, 6 μl (about 400 μg of total protein, in case of Coomassie staining), or 1,5 μl (about 100 μg of total protein, in case of Sypro staining) of each plasma sample were heated for 5' at 95°C with 10 μl of SDS 5% w/v, DTT 2.5% w/v, and diluted to 330 μl with 7 M urea, 2 M thiourea, 4% w/v CHAPS, 0.5% v/v of IPG buffer 3-10 NL, and traces of bromophenol blue, as in Hughes et al., 1992. The sample was then loaded on 18 cm IPG 3-10 NL strips by in-gel rehydration (2 h at 0 V, 12 h at 30 V). Isoelectrofocusing was then performed at 20°C on an
IPGphor apparatus (GE Healthcare) or equivalent as follows: 500 V at 500 V/hr, 1,000 V at 1,000 V/hr with a linear gradient; 8,000 V at 13,500 V/hr with a linear gradient, 8,000 V at 72,000 V/hr. Prior to SDS-PAGE, the IPG strips were equilibrated twice for 15' in a buffer containing 50 mM Tris-HCl pH 8.8, 6 M urea, 30% v/v glycerol, 2% w/v SDS, and traces of bromophenol blue containing 1% w/v DTT for the first equilibration step, and 2.5% w/v iodoacetamide for the second one. SDS-PAGE was performed on 12.5% polyacrylamide gels (1.5 mm thick) according to Laemmli [1970] but without stacking gel, using a Hoefer SE600 apparatus (GE Healthcare) or equivalent apparatus. The second dimension was run at 60 mA/gel at 16°C until the bromophenol blue dye front reached the bottom of the gel. Standard proteins having MW (15-100 kDa) and pI (pH 4,5-8,5) may be used for the calibration of the MW and of the pI. The gels must then be stained with Coomassie brilliant blue R350 (Sigma) or with Sypro Ruby (Molecular Probes, Invitrogen). After staining, the digital images of the gels are acquired by using an ImageMaster Labscan V3.0 scanner (GE Healthcare) or an equivalent for the gels stained with Coomassie or a CCD camera (Perkin Elmer) for gel stained with Sypro, and the images are analysed with the ImageMaster 2-DE Platinum 5.0 software (GE Healthcare) or an equivalent. To identify the diagnostic spots on the tested gel, the image of the gel is overlapped with the appropriate reference gel (Figure 2). The %Vol is obtained for each diagnostic spot by densitometric analysis as a percentage ratio of the normalised density of the spot on the total of the density of all of the spots aligned between the examined gel and the reference gel.

To carry out the 2DE Western blot experiments, the plasma proteins were denatured and subsequently separated on a 2DE gel, as disclosed for Coomassie and Sypro staining. After the gels were run, they were immediately introduced into an aqueous solution containing 25 mM Tris, 40 mM 6-aminohexanoic acid and 20% v/v methanol, checking that the final pH was 9.4. The proteins separated thereby were transferred to a nitrocellulose membrane (Hybond C-extra with 0.45 micrometre pores; GE Healthcare, Uppsala, Sweden) by applying a "semi-dry" transfer. After transfer, the membranes were incubated for 1 hour at 42°C in a blocking solution containing TBS and 0.1% w/v Tween 20 (T-TBS) and 3% w/v fish gelatine. T-TBS was also used for washing away unspecific antibody binding. A polyclonal ALB(N17) Santa Cruz antibody was used as a primary recognition antibody at a dilution of 1:500. As the antibody is raised in goat, an anti-goat HRP (horse radish peroxidase) conjugate was used as secondary detection antibody; the membrane was therefore incubated with a specific chemiluminescent substrate provided by the ECL Western Blotting kit (Pierce, Euroclone). The images corresponding to the proteins identified after film exposure, were acquired by an ImageMaster Labscan V3.0 (GE Healthcare, Uppsala, Sweden).

2) ALS DIAGNOSIS

(2.1). Computation

The following diagnostic function DF may be computed from the %Vol of the spots identified in Figure 1:

$$DF = -3.349\ \%Vol(182) + 8.688\ \%Vol(183) - 1.146\ \%Vol(65) + 5.536\ \%Vol(110) + 1.652\ \%Vol(87) + 2.630\ \%Vol(125) + 3.026\ \%Vol(34) - 2.426\ \%Vol(66) + 30.098\ \%Vol(8) - 2.38$$

wherein it should be understood that the numbering of each spot shown highlighted by way of example on the 2DE map in Figure 1 corresponds to the SEQ ID NO shown in Figure 1A for each spot, i.e.:

Spot 8 and spot 66 = SEQ ID NO 1;
spot 110 = SEQ ID NO 2;
spot 65 = SEQ ID NO 3;
spot 34 = SEQ ID NO 4;
spot 125 = SEQ ID NO 5;
spot 182 = SEQ ID NO 6;
spot 183 = SEQ ID NO 7;
spot 87 = SEQ ID NO 8;

It should also be understood that any other function (for example a different linear function or a non-linear function) of the indicated amounts of biomarkers obtained as disclosed or by Western Blot, ELISA or other methods, may be used instead of the disclosed function, and falls within the scope of the present patent.

(2.2). Evaluation of the results

As shown in Figure 6, the value of DF tends to be positive in the presence of ALS. It is therefore assumed that, in case the quantification of the suggested biomarkers leads to a value of DF > 0, the tested individual suffers from ALS.

The person skilled in the art will have no difficulty in recognising that, if a different function is used, a different threshold value must be selected, but the information inputted, however related to one or more of the reported biomarkers, is the same and is covered by the present patent.

(3) PROGRESSION OF THE DISEASE

(3.1). Computation

To identify spot 101 on the test gel, the same gel is overlapped to the image of a reference gel (Figure 9). The %Vol of spot 101 is therefore computed as previously disclosed.

(3.2). Evaluation

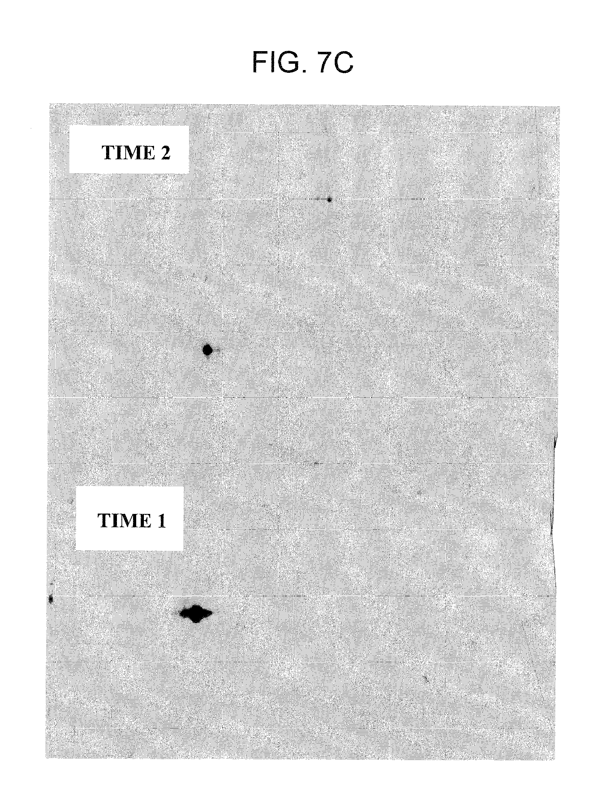

In the course of time from the clinical onset of the disease, the %Vol of spot 101 is expected to decrease in ALS patients (Figure 7A, 7B and 7C). Therefore, a comparison of the %Vol of this spot with the corresponding value obtained in a previous moment is informative of the progression of the disease. As is apparent to the person skilled in the art, the measurement of the amount of protein corresponding to spot 101 (C3c) by any other means may replace the evaluation of the %Vol of spot 101, without departing from the scope of the present patent. The decrease of %Vol of spot 101 with the progression of the disease has also been studied by 2DE-Western blotting as disclosed hereinafter (Figure 7C).

To carry out the 2DE Western blot experiments, the plasma proteins were denatured and subsequently separated on a 2DE gel, as disclosed for Coomassie and Sypro staining. After the gels were run, they were immediately introduced into an aqueous solution containing 25 mM Tris, 40 mM 6-aminohexanoic acid and 20% v/v methanol, checking that the final pH was 9.4. The proteins separated thereby were transferred to a nitrocellulose membrane (Hybond C-extra with 0.45 micrometre pores; GE Healthcare, Uppsala, sweden) by applying a "semi-dry" transfer. After transfer, the membranes were incubated for 1 hour at 42°C in a blocking solution containing TBS and 0.1% w/v Tween 20 (T-TBS) and 5% w/v milk. T-TBS was used for washing away unspecific antibody binding. A polyclonal antibody (A 0062, DAKO) was used as a primary recognition antibody at a dilution of 1:5000. As it was raised in rabbit, an anti-rabbit HRP (horse radish peroxidase) conjugate was used as secondary detection antibody; the membrane was then incubated with a specific chemiluminescent substrate provided by the ECL Western Blotting kit (Pierce, Euroclone). The images corresponding to the proteins identified after film exposure were acquired by an ImageMaster Labscan V3.0 (GE Healthcare, Uppsala, Sweden).

Advantages

The person skilled in the art and dealing with ALS will immediately recognise the advantages of a diagnostic test such as that disclosed and of the corresponding biomarkers, which have the following advantages:

1. greater objectivity with respect to clinical diagnostic methods, the test being related to a molecular aspect of the disease and to the measurement of quantitative parameters for the diagnosis;
2. greater accuracy of the suggested biomarkers with respect to others, as they are selected by considering two control groups, the first formed by healthy subjects and the second by cardiovascular subjects, to distinguish between specific ALS markers and generic inflammation markers;
3. simple sampling required for diagnosis, as the measurement is based on haematic biomarkers, small volumes of blood and on a single value for the diagnosis of ALS;
4. possibility of developing simplified diagnostic methodologies, as the detected biomarkers may be detected with techniques other than 2DE;
5. possibility of a follow-up at a quantitative level of the disease and of the therapies, as one of the detected biomarkers varies its level during the course of the disease.

Variants

What has been disclosed up to this point is a privileged example of the invention with some possible variations. The terms, descriptions and figures are shown by mere way of illustration and do not imply limitations in the aims or object of the present patent. The person skilled in the art will recognise that many possible variants are possible in the spirit and scope of the present invention, in the description of which each term has been used in the broadest sense possible, without any limitation unless explicitly indicated.

In particular, the present invention is based on the discovery of a set of ALS biomarkers, the amount of which is correlated to the presence of the disease (all of the biomarkers indicated in Figure 1A, i.e. all except C3c) or to its progression (only C3c, Figure 1B). The amount of these biomarkers may be evaluated by 2DE, as previously disclosed, but it is clear to a person skilled in the art that any other evaluation method of the level of one or more of the biomarkers shown in Figure 1 falls within the scope and spirit of the present patent application. By mere way of example, the biomarkers may be quantified by one or more of the following alternative techniques:

1. Western blot
2. ELISA
3. HPLC
4. Mass spectrometry.

Furthermore, a variant falling within the scope of the present patent application consists in using any numerical combination of the amount of some or all of the disclosed biomarkers to compute a different diagnostic (linear or non-linear) function or derive any statistical parameter that provides a score useful for the diagnosis of ALS or for the evaluation of its progression.

It should also be understood that any combination of the present biomarkers with other diagnostic methods for ALS or other neurological disorders must be considered to be part of the present patent application.

It should finally be understood that, although the use of human blood samples is preferable as compared to other biological material, the testing and use of any combination of biomarkers shown in Figure 1 in biological samples other than human blood must be part of the present patent application.

References

Adams RA, Passino M, Sachs BD, Nuriel T, Akassoglou K. (2004). Fibrin mechanisms and functions in nervous system pathology. Mol Interv, 4:163.

Aivado M, Spentzos D, Germing U, Alterovitz G, Meng XY, Grall F, Giagounidis AA, Klement G, Steidl U, Otu HH, Czibere A, Prall WC, Iking-Konert C, Shayne M, Ramoni MF, Gattermann N, Haas R, Mitsiades CS, Fung ET, Libermann TA. (2007). Serum proteome profiling detects myelodysplastic syndromes and identifies CXC chemokine ligands 4 and 7 as markers for advanced disease. Proc Natl Acad Sci USA, 104:1307.

Akassoglou K e Strickland S. (2002). Nervous system pathology: the fibrin perspective. Biol Chem, 383:37.

Avasarala JR, Wall MR, Wolfe GM. (2005). A distinctive molecular signature of multiple sclerosis derived from MALDI-TOF/MS and serum proteomic pattern analysis: detection of three biomarkers. J Mol Neurosci, 25:119.

Barber SC, Mead RJ, Shaw PJ. (2006). Oxidative stress in ALS: a mechanism of neurodegeneration and a therapeutic target. Biochimica et Biophysica Acta, 1762:1051.

Belsh JM. (1996). Epidemiology and Historical Perspective of ALS. In: Belsh JM, Schiffmann PL (eds) Amyotrophic Lateral Sclerosis - Diagnosis and Management for the Clinician. Futura Publishing Company, 3.

Bito R, Hino S, Baba A, Tanaka M, Watabe H, Kawabata H. (2005). Degradation of oxidative stress-induced denatured albumin in rat liver endothelial cells. Am J Physiol Cell Physiol, 289:C531.

Bowser R, Cudkowicz M, Kaddurah-Daouk R. (2006). Biomarkers for amyotrophic lateral sclerosis. Expert Rev Mol Diagn, 6:387.

Brettschneider J, Mogel H, Lehmensiek V, Ahlert T, Süssmuth S, Ludolph AC, Tumani H. (2008). Proteome analysis of cerebrospinal fluid in amyotrophic lateral sclerosis (ALS). Neurochem Res, 2008 May 15. [Epub ahead of print]

Bruijn LI, Miller TM, Cleveland DW. (2004). Unraveling the mechanisms involved in motor neuron degeneration in ALS. Annu Rev Neurosci, 27: 723.

Cohen J. (1992). A power primer. Psychological Bulletin, 112:155.

Couratier P, Yi FH, Preud'homme JL, Clavelou P, White A, Sindou P, Vallat JM, Jauberteau MO (1998). Serum autoantibodies to neurofilament proteins in sporadic amyotrophic lateral sclerosis. J Neurol Sci, 154:137.

Danzeisen R, Achsel T, Bederke U, Cozzolino M, Crosio C, Ferri A, Frenzel M, Gralla EB, Huber L, Ludolph A, Nencini M, Rotilio G, Valentine JS, Carri MT. (2006). Superoxide dismutase 1 modulates expression of transferrin receptor. J Biol Inorg Chem, 11:489.

Demestre M, Howard RS, Orrell RW, Pullen AH. (2006). Serine proteases purified from sera of patients with amyotrophic lateral sclerosis (ALS) induce contrasting cytopathology in murine motoneurones to IgG. Neuropathol Appl Neurobiol, 32:141.

Doran P, Martin G, Dowling P, Jockusch H, Ohlendieck K. (2006). Proteome analysis of the dystrophin-deficient MDX diaphragm reveals a drastic increase in the heat shock protein cvHSP. Proteomics, 6:4610.

Finehout EJ, Franck Z, Choe LH, Relkin N, Lee KH. (2007). Cerebrospinal fluid proteomic biomarkers for Alzheimer's disease. Ann Neurol, 61:120.

Funding M, Vorum H, Honoré B, Nexø E, Ehlers N. (2005). Proteomic analysis of aqueous humour from patients with acute corneal rejection. Acta Ophthalmol Scand, 83:31.

German DC, Gurnani P, Nandi A, Garner HR, Fisher W, Diaz-Arrastia R, O'Suilleabhain P, Rosenblatt KP. (2007). Serum biomarkers for Alzheimer's disease: proteomic discovery. Biomed Pharmacother, 61:383.

Goldknopf IL, Sheta EA, Bryson J, Folsom B, Wilson C, Duty J, Yen AA, Appel SH. (2006). Complement C3c and related protein biomarkers in amyotrophic lateral sclerosis and Parkinson's disease. Biochem Biophys Res Commun, 342:1034.

Grewal RP, Morgan TE, Finch CE. (1999). ClqB and clusterin mRNA increase in association with neurodegeneration in sporadic amyotrophic lateral sclerosis. Neurosci Lett, 271:65.

Hedges L e Olkin I, editori. (1985). Statistical Methods for Meta-Analysis. New York: Academic Press.

Holland PC, Hancock SW, Hodge D, Thompson D, Shires S, Evans S. (2001). Degradation of albumin in meningococcal sepsis. Lancet, 357:2102.

Hughes GJ, Frutiger S, Paquet N, Ravier F, Pasquali C, Sanchez JC, James R, Tissot JD, Bjellqvist B, Hochstrasser DF. (1992). Plasma protein map: an update by microsequencing. Electrophoresis, 13:707.

Ilzecka J, Stelmasiak Z, Dobosz B. (2001). Interleukin-lbeta converting enzyme/Caspase-1 (ICE/Caspase-1) and soluble APO-1/Fas/CD 95 receptor in amyotrophic lateral sclerosis patients. Acta Neurol Scand, 103:255.

Jacobs DI, van Rijssen MS, van der Heijden R, Verpoorte R. (2001). Sequential solubilization of proteins precipitated with trichloroacetic acid in acetone from cultured Catharanthus roseus cells yields 52% more spots after two-dimensional electrophoresis. Proteomics, 1:1345.

Jacquier A, Buhler E, Schäfer MK, Bohl D, Blanchard S, Beclin C, Haase G. (2006). Alsin/Rac1 signaling controls survival and growth of spinal motoneurons. Ann Neurol, 60:105.

Julien JP. (2001). Amyotrophic lateral sclerosis: unfolding the toxicity of the misfolded. Cell, 104:581.

Kaiser T, Kamal H, Rank A, Kolb HJ, Holler E, Ganser A, Hertenstein B, Mischak H, Weissinger EM. (2004). Proteomics applied to the clinical follow-up of patients after allogeneic hematopoietic stem cell transplantation. Blood, 104:340.

Kawakami T, Hoshida Y, Kanai F, Tanaka Y, Tateishi K, Ikenoue T, Obi S, Sato S, Teratani T, Shiina S, Kawabe T, Suzuki T, Hatano N, Taniguchi H, Omata M. (2005). Proteomic analysis of sera from hepatocellular carcinoma patients after radiofrequency ablation treatment. Proteomics, 5:4287.

Kim HJ, Cho EH, Yoo JH, Kim PK, Shin JS, Kim MR, Kim CW. (2007). Proteome analysis of serum from type 2 diabetics with nephropathy. J Proteome Res, 6:735.

Lacomblez L, Bensimon G, Leigh PN, Guillet P, Meininger V. (1996). Dose-ranging study of riluzole in amyotrophic lateral sclerosis. Amyotrophic Lateral Sclerosis/Riluzole Study Group II. Lancet, 347:1425.

Laemmli UK. (1970). Cleavage of structural proteins during the assembly of the head of bacteriophage T4. Nature, 227:680.

Lee JW, Namkoong H, Kim HK, Kim S, Hwang DW, Na HR, Ha SA, Kim JR, Kim JW. (2007). Fibrinogen gamma-A chain precursor in CSF: a candidate biomarker for Alzheimer's disease. BMC Neurol, 7:14.

Li SQ, Yun J, Xue FB, Bai CQ, Yang SG, Que HP, Zhao X, Wu Z, Wang Y, Liu SJ. (2007). Comparative proteome analysis of serum from acute pulmonary embolism rat model for biomarker discovery. J Proteome Res, 6:150.

Miller RG, Mitchell JD, Lyon M, Moore DH. (2002). Riluzole for amyotrophic lateral sclerosis (ALS)/motor neuron disease (MND). Cochrane Database Syst Rev, 2:CD001447.

Mizuno Y, Amari M, Takatama M, Aizawa H, Mihara B, Okamoto K. (2006). Transferrin localizes in Bunina bodies in amyotrophic lateral sclerosis. Acta Neuropathol, 112:597.

Mizutani K, Oka N, Kusunoki S, Kaji R, Kanda M, Akiguchi I, Shibasaki H. (2003). Amyotrophic lateral sclerosis with IgM antibody against gangliosides GM2 and GD2. Intern Med, 42:277.

Moser KV e Humpel C. (2007). Blood-derived serum albumin contributes to neurodegeneration via astroglial stress fiber formation. Pharmacology, 80:286.

Perluigi M, Poon HF, Hensley K, Pierce WM, Klein JB, Calabrese V, De Marco C, Butterfield DA. (2005). Proteomic analysis of 4-hydroxy-2-nonenal-modified proteins in G93A-SOD1 transgenic mice-A model of familial amyotrophic lateral sclerosis. Free Radic Biol Med, 38:960.

Pestronk A. (1991). Motor neuropathies, motor neuron disorders, and antiglycolipid antibodies. Muscle Nerve, 14:927.

Rowland LP. (1998). Diagnosis of amyotrophic lateral sclerosis. J Neurol Sci, 160:S6.

Shaw PJ e Williams R. (2000). Serum and cerebrospinal fluid biochemical markers of ALS. Amyotroph Lateral Scler Other Motor Neuron Disord, 1:S61.

Walgren JL, Mitchell MD, Whiteley LO, Thompson DC. (2007). Evaluation of two novel peptide safety markers for exocrine pancreatic toxicity. Toxicol Sci, 96:184.

Yagame M, Suzuki D, Jinde K, Yano N, Naka R, Abe Y, Nomoto Y, Sakai H, Suzuki H, Ohashi Y. (1995). Urinary albumin fragments as a new clinical parameter for the early detection of diabetic nephropathy. Intern Med, 34:463.

Zeman D, Adam P, Kalistová H, Sobek O, Kelbich P, Andel J, Andel M. (2000). Transferrin in patients with multiple sclerosis: a comparison among various subgroups of multiple sclerosis patients. Acta Neurol Scand, 101:89.

## Claims

**1.** A polypeptide consisting of SEQ ID NO: 9 or SEQ ID NO: 10.

**2.** A pharmaceutical composition comprising a polypeptide according to claims 1.

**3.** A polypeptide comprising SEQ ID NO:9 or SEQ ID NO:10 or a composition comprising a polypeptide comprising SEQ ID NO:9 or SEQ ID NO:10 for use in a method for detecting the progression of a neurodegenerative disorder in an individual.

**4.** A polipeptide or a composition according to claim 3, wherein said neurodegenerative disorder is amyotrophic lateral sclerosis.

**5.** A polipeptide or a composition according to claim 3 or 4, wherein said polypeptide is used in combination with at least another biomarker.

**6.** A method for detecting the progression of a neurodegenerative disorder in an individual comprising the steps of:

- quantifying the level of one or more polypeptides comprising SEQ ID NO: 9 or SEQ ID NO:10 or a composition comprising one or more polypeptides comprising SEQ ID NO:9 or SEQ ID NO:10 in an isolated biological sample selected from the group consisting of blood, plasma, serum;
- comparing said level with a reference level.

**7.** The method according to claim 6, wherein said neurodegenerative disorder is amyotrophic lateral sclerosis.

**8.** The method according to claim 6 or 7, wherein the quantification of said one or more polypeptides or of said composition is performed by means of a technique selected from the group consisting of two-dimensional electrophoresis, densitometry, Western blotting, ELISA, HPLC, mass spectrometry and protein chips.

FIG. 1A

**ALS diagnostic spots**

| Seq. ID NO | Spot | LC-ESI MS/MS Identification | Accession number | N. of peptides | Covered Sequence | Apparent pI | Apparent MW | MS score |
|---|---|---|---|---|---|---|---|---|
| 1 | 8 | **Albumin fragment** | gi 28592 | 15 | 37-298 | ~6,0 | ~30 kDa | 579 |
| 1 | 66 | **Albumin fragment** | gi 28592 | 14 | 37-298 | ~5,5 | ~30 kDa | 743 |
| 2 | 110 | **Albumin fragment** | gi 28592 | 20 | 37-413 | ~5,0-5,5 | ~45 kDa | 1039 |
| 3 | 65 | **Albumin fragment** | gi 28592 | 19 | 37-310 | ~6,0 | ~30 kDa | 985 |
| 4 | 34 | **Albumin fragment** | gi 28592 | 8 | 500-609 | ~6,0-6,5 | ~18 kDa | 561 |
| 5 | 125 | **Gamma fibrinogen** | gi 223170 | 11 | 63-256 | ~5,0-5,5 | ~50kD | 571 |
| 6 | 182 | **Serotransferrin** | gi 4557871 | 28 | 27-676 | ~6,0 | ~75 kDa | 1466 |
| 7 | 183 | IgM | gi33873884 | 16 | 211-537 | ~6,0 | ~75 kDa | 720 |
| 8 | 87 | **Clusterin** | gi 178855 | 8 | 12-404 | ~5,0 | ~35 kDa | 421 |

FIG. 1B

**Spot for evaluating the progression of ALS**

| Seq. ID | Spot | LC-ESI MS/MS Identification | Accession number | N. of peptides | Covered sequence | Apparent pI | Apparent MW | MS score |
|---|---|---|---|---|---|---|---|---|
| 9 | 101 | C3 complement | gi 78101271 | 19 | 6-324 | ~5,0 | ~40 kDa | 1057 |

# FIG. 3

| SEQ ID NO | SPOT | %Vol (average) +/- SD | | | p | | |
|---|---|---|---|---|---|---|---|
| | | CTR | ALS | INF | CTR vs INF | CTR vs ALS | ALS vs INF |
| 1 | 8 | 0.005 ± 0.010 | 0.027 ± 0.024 | 0 | NS | ** | ** |
| 1 | 66 | 0.089 ± 0.043 | 0.196 ± 0.131 | 0.128 ± 0.108 | NS | * | NS |
| 2 | 110 | 0.070 ± 0.031 | 0.155 ± 0.076 | 0.081 ± 0.060 | NS | *** | * |
| 3 | 65 | 0.144 ± 0.061 | 0.275 ± 0.193 | 0.120 ± 0.111 | NS | NS | ** |
| 4 | 34 | 0.064 ± 0.030 | 0.162 ± 0.071 | 0.134 ± 0.136 | NS | *** | NS |
| 5 | 125 | 0.207 ± 0.070 | 0.301 ± 0.171 | 0.094 ± 0.064 | * | NS | *** |
| 6 | 87 | 0.050 ± 0.021 | 0.145 ± 0.096 | 0.081 ± 0.040 | NS | *** | NS |
| 7 | 182 | 0.165 ± 0.096 | 0.407± 0.185 | 0.351 ± 0.300 | NS | *** | NS |
| 8 | 183 | 0.087 ± 0.066 | 0.256 ± 0.129 | 0.171 ± 0.122 | NS | *** | NS |

***Note*: The values are expressed as values of the relative volumes (%Vol) +/- standard deviation (SD). The statistical analysis of the differentially expressed spots is described by the values of p and is diagrammatically represented as follows:**

NS $= p \geq 0.05$;

* $= p < 0.05$;

** $= p < 0.01$;

*** $= p < 0.001$

FIG. 4A

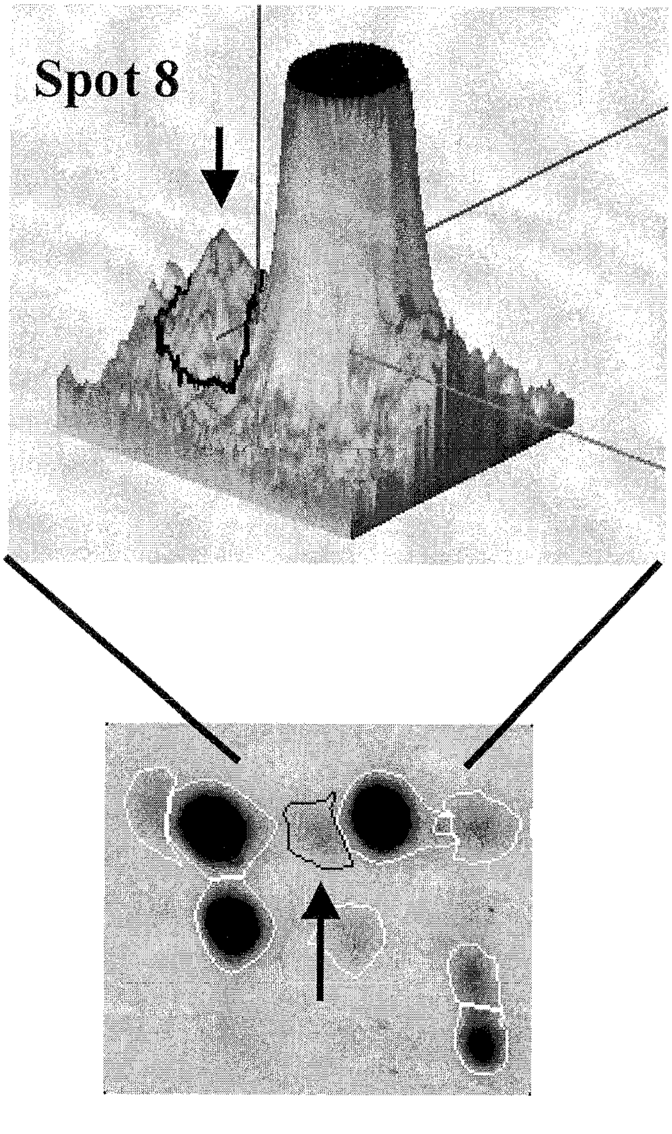

FIG. 4B

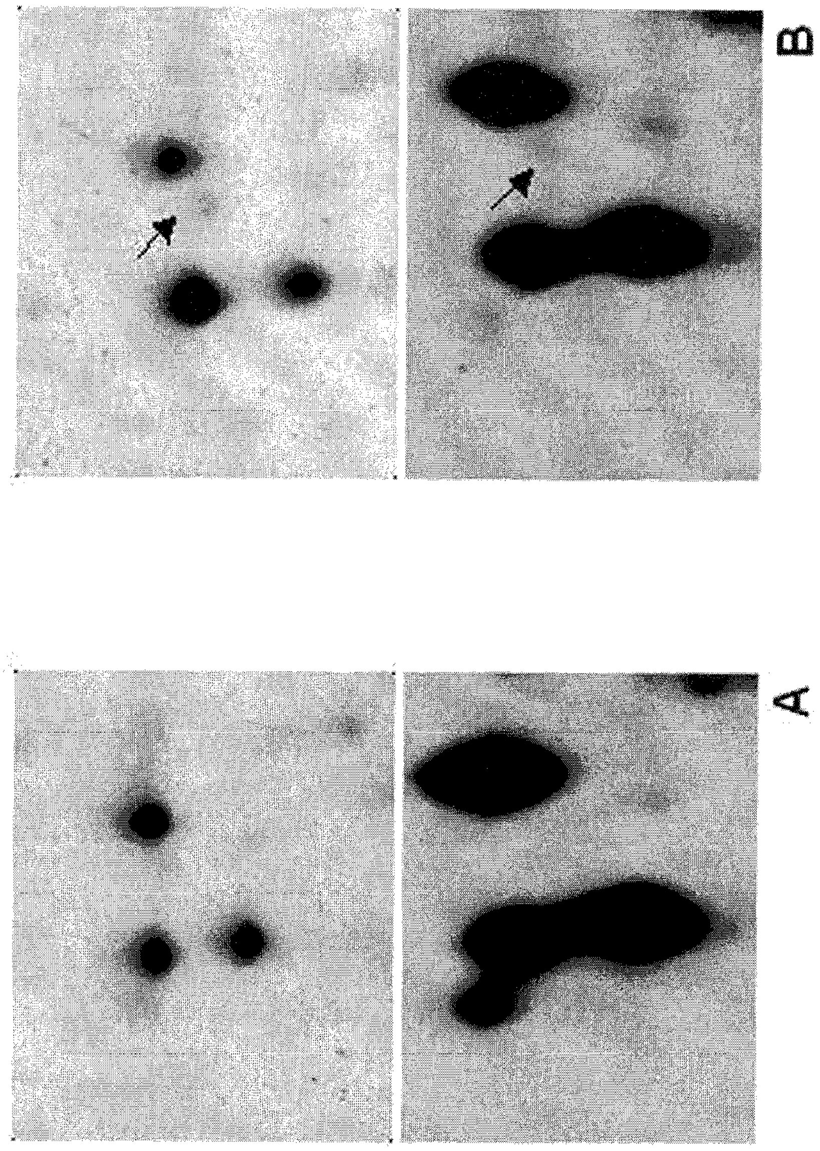

FIG. 5

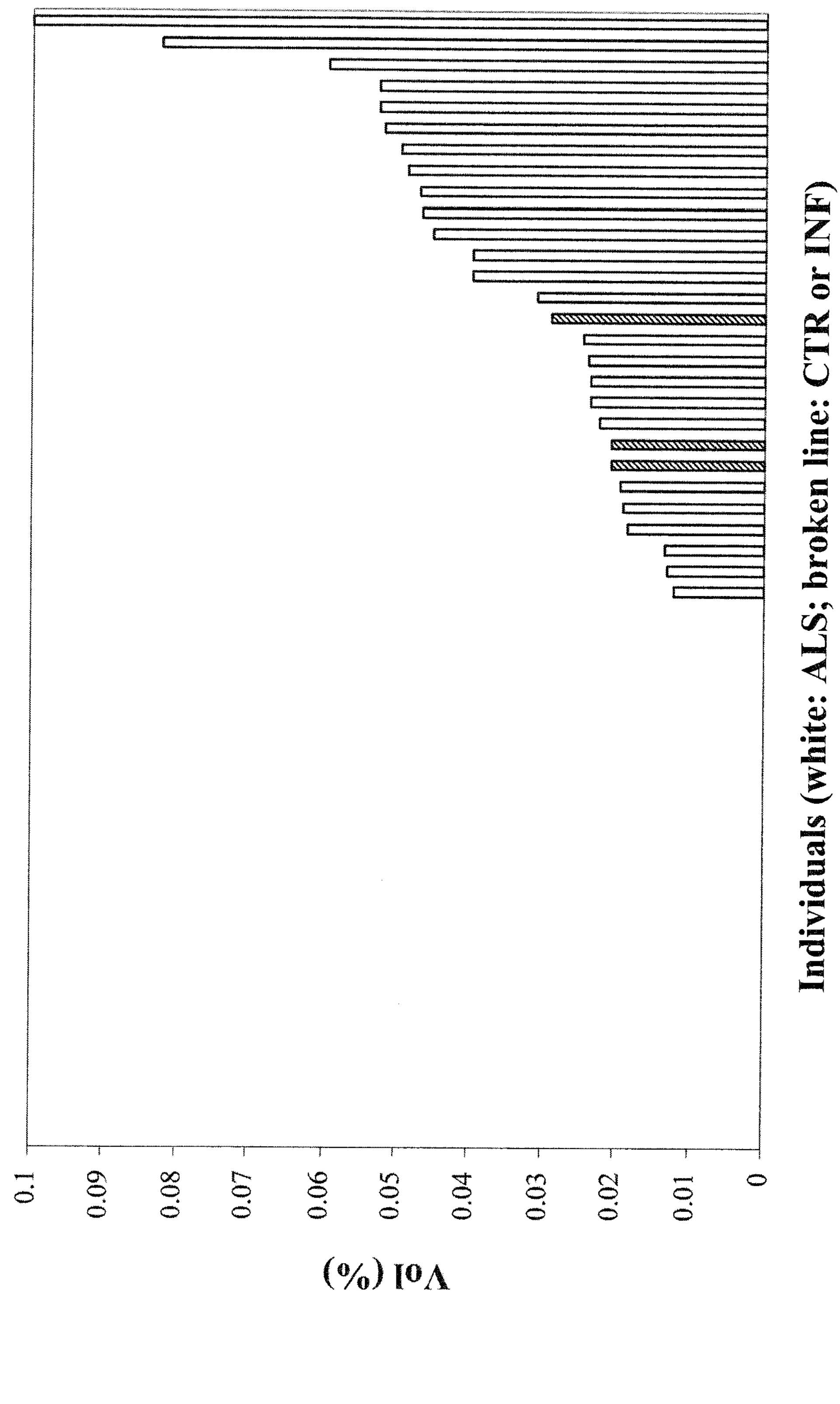
Vol (%)
0.1
0.09
0.08
0.07
0.06
0.05
0.04
0.03
0.02
0.01
0
Individuals (white: ALS; broken line: CTR or INF)

FIG. 6

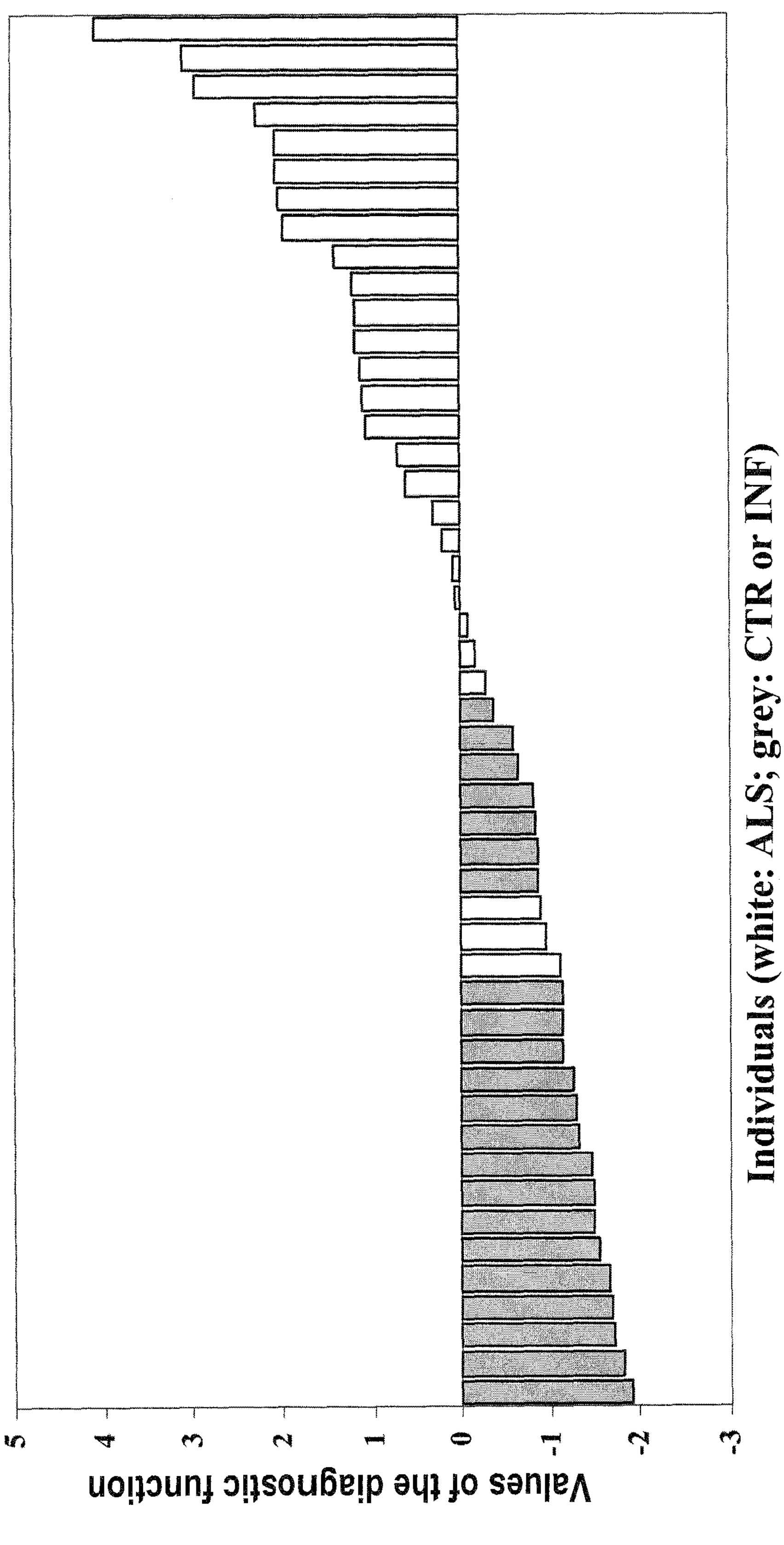
Values of the diagnostic function
5
4
3
2
1
0
-1
-2
-3
Individuals (white: ALS; grey: CTR or INF)

FIG. 7A

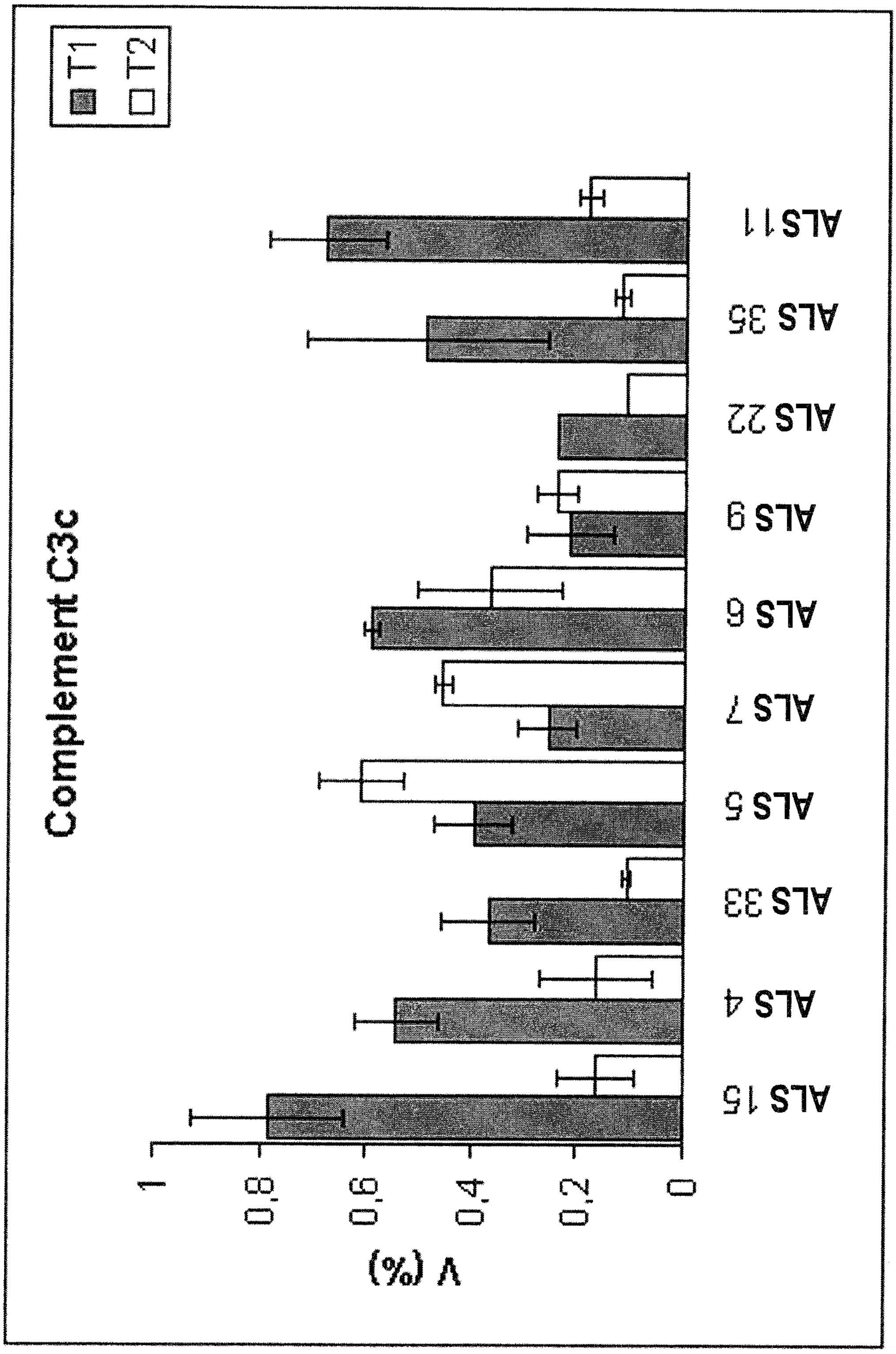
Complement C3c
T1
T2
V (%)
1
0,8
0,6
0,4
0,2
0
ALS 15
ALS 4
ALS 33
ALS 5
ALS 7
ALS 6
ALS 9
ALS 22
ALS 35
ALS 11

FIG. 7B

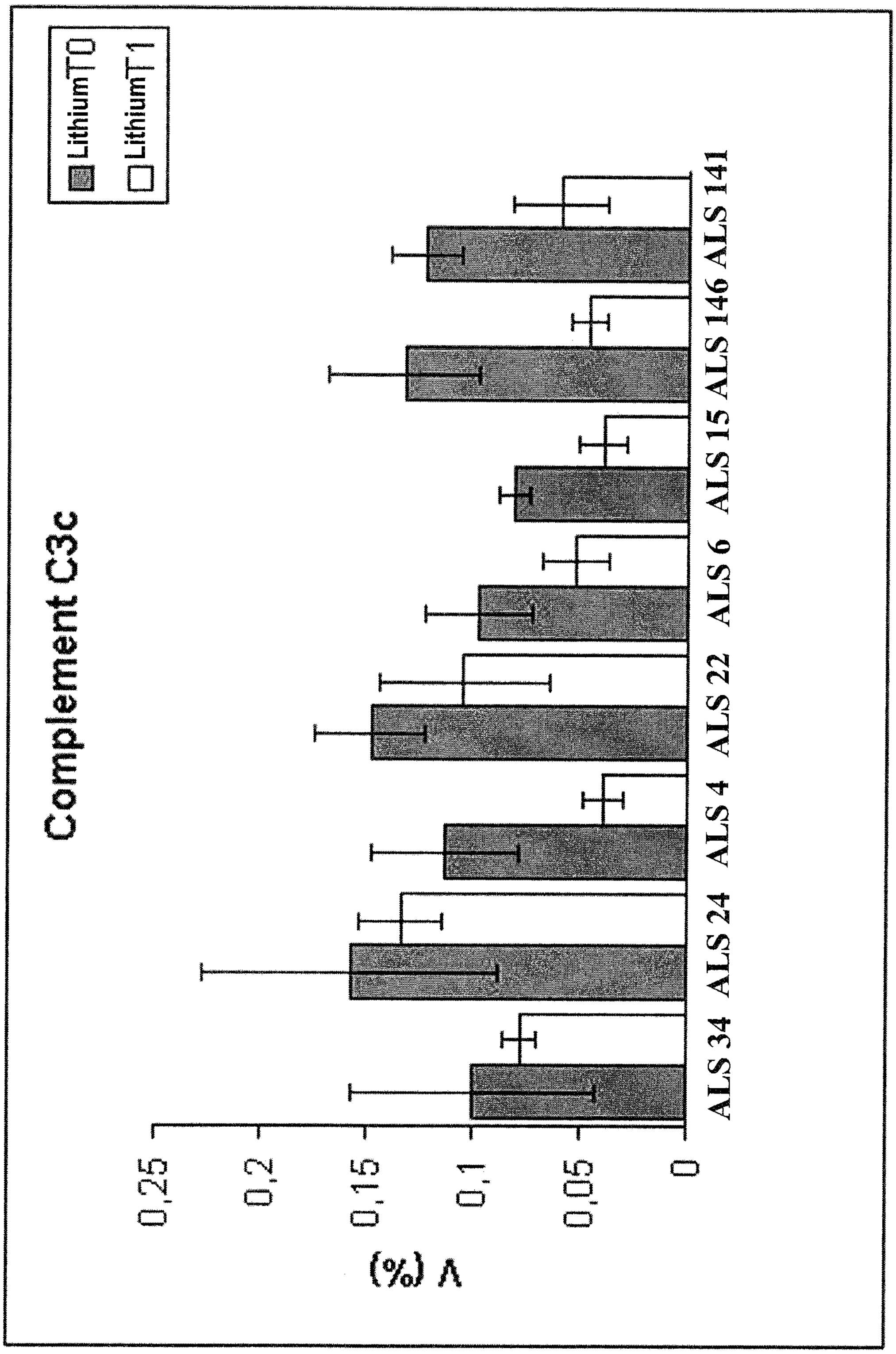
Complement C3c
LithiumT0
LithiumT1
V (%)
0,25
0,2
0,15
0,1
0,05
0
ALS 34
ALS 24
ALS 4
ALS 22
ALS 6
ALS 15
ALS 146
ALS 141

FIG. 8

```
1                                        40                                      80                                      120
|                                        |                                       |                                       |
          SEQ ID NO 3      dlgeenfkalvliafaqylqqcpfedhvklvnevtefaktcvadesaencdkslhtlfgdklctvatlretygemadccakqep
          SEQ ID NO 2                                   LVNEVTEFAKTCVADESAENCDKSLHTLFGDKLCTVATLRETYGEMADCCAKQEP
          SEQ ID NO 1      dlgeenfkalvliafaqylqqcpfedhvklvnevtefaktcvadesaencdkslhtlfgdklctvatlretygemadccakqep

MKWVTFISLLFLFSSAYSRGVFRRDAHKSEVAHRFKDLGEENFKALVLIAFAQYLQQCPFEDHVKLVNEVTEFAKTCVADESAENCDKSLHTLFGDKLCTVATLRETYGEMADCCAKQEP

          140                                     180                                     220
          |                                       |                                       |
ernecflqhkddnpnlprlvrpevdvmctafhdneetflkkylyeiarrhpyfyapellffakrykaaftecccqaadkaacllpkldelrdegkassakqrlkcaslqkfgerafkawav
ERNECFLQHKDDNPNLPRLVRPEVDVMCTAFHDNEETFLKKYLYEIARRHPYFYAPELLFFAKRYKAAFTECCQAADKAACLLPKLDELRDEGKASSAKQRLKCASLQKFGERAFKAWAV
ernecflqhkddnpnlprlvrpevdvmctafhdneetflkkylyeiarrhpyfyapellffakrykaaftecccqaadkaacllpkldelrdegkassakqrlkcaslqkfgerafkawav

ERNECFLQHKDDNPNLPRLVRPEVDVMCTAFHDNEETFLKKYLYEIARRHPYFYAPELLFFAKRYKAAFTECCQAADKAACLLPKLDELRDEGKASSAKQRLKCASLQKFGERAFKAWAV

         250                                     290                                     330
         |                                       |                                       |
arlsqrfpkaefaevsklvtdltkvhtecchgdllecaddradlakyicenqdsisskl kecccekpllek
ARLSQRFPKAEFAEVSKLVTDLTKVHTECCHGDLLECADDRADLAKYICENQDSISSKLKECCEKPLLEKSHCIAEVENDEMPADLPSLAADFVESKDVCKNYAEAKDVFLGMFLYEYAR
arlsqrfpkaefaevsklvtdltkvhtecchgdllecaddradlakyicenqdsissk

ARLSQRFPKAEFAEVSKLVTDLTKVHTECCHGDLLECADDRADLAKYICENQDSISSKLKECCEKPLLEKSHCIAEVENDEMPADLPSLAADFVESKDVCKNYAEAKDVFLGMFLYEYAR

         370                                     410                                     450
         |                                       |                                       |
RHPDYSVVLLLRLAKTYKTTLEKCCAAADPHECYAKVFDEFKPLVEEPQNLIK          SEQ ID NO 2

RHPDYSVVLLLRLAKTYKTTLEKCCAAADPHECYAKVFDEFKPLVEEPQNLIKQNCELFEQLGEYKFQNALLVRYTKKVPQVSTPTLVEVSRNLGKVGSKCCKHPEAKRMPCAEDYLSVV

         490                                     530                                          581
         |                                       |                                            |
SEQ ID NO 4      cctesl vnrrpcfsalevdetyvpkefnaetftfhadictlsekerqikkqtalvelvkhkpkatkeqlkavmddfaafvekcckaddketcfaeegkklv

LNQLCVLHEKTPVSDRVTKCCTESLVNRRPCFSALEVDETYVPKEFNAETFTFHADICTLSEKERQIKKQTALVELVKHKPKATKEQLKAVMDDFAAFVEKCCKADDKETCFAEEGKKLV

        609
        |
aasqaalgl        SEQ ID NO 4

AASQAALGL
```

**Human albumin**, Accession number: 28592

Europäisches Patentamt
European Patent Office
Office européen des brevets

# EUROPEAN SEARCH REPORT

Application Number
EP 11 19 3436

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PASINETTI G M; UNGAR L H; LANGE D J; YEMUL S; DENG H; YUAN X; BROWN R H; CUDKOWICZ M E; NEWHALL K; PESKIND E; MARCUS S; HO L: "Identification of potential CSF biomarkers in ALS.", NEUROLOGY 25 APR 2006, vol. 66, no. 8, 25 April 2006 (2006-04-25), pages 1218-1222, XP002576556, ISSN: 1526-632X<br>* page 1219, right-hand column, paragraph 3 - page 1220, left-hand column, paragraph 1; figure 3 *<br>* page 1221, left-hand column, paragraph 4 - right-hand column, paragraph 1 *<br>----- | 1-8 | INV.<br>C07K14/47<br>G01N33/68 |
| X | WO 2006/035237 A2 (PROTEOME SCIENCES PLC [GB]; KING S COLLEGE LONDON [GB]; WESTBROOK JULE) 6 April 2006 (2006-04-06)<br>* page 22, last paragraph - page 23, paragraph 1; claims 36,39; figure 12 *<br>----- | 3-6,8 | |
| X,P | US 2008/289964 A1 (GOLDKNOPF IRA LEONARD [US] ET AL) 27 November 2008 (2008-11-27)<br>* the whole document *<br>----- | 1-8 | TECHNICAL FIELDS SEARCHED (IPC)<br>C07K<br>G01N |
| A | DONNENFELD H ET AL: "Deposits of IgG and C3 in the spinal cord and motor cortex of ALS patients", JOURNAL OF NEUROIMMUNOLOGY, ELSEVIER SCIENCE PUBLISHERS BV, XX, vol. 6, no. 1, 1 February 1984 (1984-02-01), pages 51-57, XP023695071, ISSN: 0165-5728, DOI: 10.1016/0165-5728(84)90042-0 [retrieved on 1984-02-01]<br>* the whole document *<br>----- | 1-8 | |

The present search report has been drawn up for all claims

| Place of search | Date of completion of the search | Examiner |
|---|---|---|
| The Hague | 21 June 2012 | Fuhr, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

3

EPO FORM 1503 03.82 (P04C01)

## ANNEX TO THE EUROPEAN SEARCH REPORT ON EUROPEAN PATENT APPLICATION NO. EP 11 19 3436

This annex lists the patent family members relating to the patent documents cited in the above-mentioned European search report.
The members are as contained in the European Patent Office EDP file on
The European Patent Office is in no way liable for these particulars which are merely given for the purpose of information.

21-06-2012

| Patent document cited in search report | Publication date | Patent family member(s) | Publication date |
|---|---|---|---|
| WO 2006035237 A2 | 06-04-2006 | AT 495445 T | 15-01-2011 |
| | | AU 2005288689 A1 | 06-04-2006 |
| | | CA 2580532 A1 | 06-04-2006 |
| | | EP 1794594 A2 | 13-06-2007 |
| | | EP 2354795 A1 | 10-08-2011 |
| | | JP 2008514946 A | 08-05-2008 |
| | | JP 2011209291 A | 20-10-2011 |
| | | US 2008070995 A1 | 20-03-2008 |
| | | US 2011165146 A1 | 07-07-2011 |
| | | WO 2006035237 A2 | 06-04-2006 |
| US 2008289964 A1 | 27-11-2008 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2008003202 A
- US 2007093443 A
- WO 06048777 A


### Non-patent literature cited in the description


- **KARLIN ; ALTSCHUL.** *Proc. Natl. Acad. Sci. USA,* 1990, vol. 87, 2264-2268
- **KARLIN ; ALTSCHUL.** *Proc. Natl. Acad. Sci. USA,* 1993, vol. 90, 5873-5877
- **ALTSCHUL et al.** *J. Mol. Biol,* 1990, vol. 215, 403-410
- **ALTSCHUL et al.** *Nucleic Acids Res.,* 1997, vol. 25, 3389-3402
- **DEVEREUX et al.** *Nucleic Acids Research,* 1984, vol. 12, 387
- **BOWIE et al.** *Science,* 1990, vol. 247, 1306-1310
- **ADAMS RA ; PASSINO M ; SACHS BD ; NURIEL T ; AKASSOGLOU K.** Fibrin mechanisms and functions in nervous system pathology. *Mol Interv,* 2004, vol. 4, 163
- **AIVADO M ; SPENTZOS D ; GERMING U ; ALTEROVITZ G ; MENG XY ; GRALL F ; GIAGOUNIDIS AA ; KLEMENT G ; STEIDL U ; OTU HH.** Serum proteome profiling detects myelodysplastic syndromes and identifies CXC chemokine ligands 4 and 7 as markers for advanced disease. *Proc Natl Acad Sci USA,* 2007, vol. 104, 1307
- **AKASSOGLOU K ; STRICKLAND S.** Nervous system pathology: the fibrin perspective. *Biol Chem,* 2002, vol. 383, 37
- **AVASARALA JR ; WALL MR ; WOLFE GM.** A distinctive molecular signature of multiple sclerosis derived from MALDI-TOF/MS and serum proteomic pattern analysis: detection of three biomarkers. *J Mol Neurosci,* 2005, vol. 25, 119
- **BARBER SC ; MEAD RJ ; SHAW PJ.** Oxidative stress in ALS: a mechanism of neurodegeneration and a therapeutic target. *Biochimica et Biophysica Acta,* 2006, vol. 1762, 1051
- Epidemiology and Historical Perspective of ALS. **BELSH JM.** Amyotrophic Lateral Sclerosis - Diagnosis and Management for the Clinician. Futura Publishing Company, 1996, 3
- **BITO R ; HINO S ; BABA A ; TANAKA M ; WATABE H ; KAWABATA H.** Degradation of oxidative stress-induced denatured albumin in rat liver endothelial cells. *Am J Physiol Cell Physiol,* 2005, vol. 289, C531
- **BOWSER R ; CUDKOWICZ M ; KADDURAH-DAOUK R.** Biomarkers for amyotrophic lateral sclerosis. *Expert Rev Mol Diagn,* 2006, vol. 6, 387
- **BRETTSCHNEIDER J ; MOGEL H ; LEHMENSIEK V ; AHLERT T ; SÜSSMUTH S ; LUDOLPH AC ; TUMANI H.** Proteome analysis of cerebrospinal fluid in amyotrophic lateral sclerosis (ALS. *Neurochem Res,* 15 May 2008
- **BRUIJN LI ; MILLER TM ; CLEVELAND DW.** Unraveling the mechanisms involved in motor neuron degeneration in ALS. *Annu Rev Neurosci,* 2004, vol. 27, 723
- **COHEN J.** A power primer. *Psychological Bulletin,* 1992, vol. 112, 155
- **COURATIER P ; YI FH ; PREUD'HOMME JL ; CLAVELOU P ; WHITE A ; SINDOU P ; VALLAT JM ; JAUBERTEAU MO.** Serum autoantibodies to neurofilament proteins in sporadic amyotrophic lateral sclerosis. *J Neurol Sci,* 1998, vol. 154, 137
- **DANZEISEN R ; ACHSEL T ; BEDERKE U ; COZZOLINO M ; CROSIO C ; FERRI A ; FRENZEL M ; GRALLA EB ; HUBER L ; LUDOLPH A.** Superoxide dismutase 1 modulates expression of transferrin receptor. *J Biol Inorg Chem,* 2006, vol. 11, 489
- **DEMESTRE M ; HOWARD RS ; ORRELL RW ; PULLEN AH.** Serine proteases purified from sera of patients with amyotrophic lateral sclerosis (ALS) induce contrasting cytopathology in murine motoneurones to IgG. *Neuropathol Appl Neurobiol,* 2006, vol. 32, 141
- **DORAN P ; MARTIN G ; DOWLING P ; JOCKUSCH H ; OHLENDIECK K.** Proteome analysis of the dystrophin-deficient MDX diaphragm reveals a drastic increase in the heat shock protein cvHSP. *Proteomics,* 2006, vol. 6, 4610

- **FINEHOUT EJ ; FRANCK Z ; CHOE LH ; RELKIN N ; LEE KH.** Cerebrospinal fluid proteomic biomarkers for Alzheimer's disease. *Ann Neurol,* 2007, vol. 61, 120
- **FUNDING M ; VORUM H ; HONORÉ B ; NEXØ E ; EHLERS N.** Proteomic analysis of aqueous humour from patients with acute corneal rejection. *Acta Ophthalmol Scand,* 2005, vol. 83, 31
- **GERMAN DC ; GURNANI P ; NANDI A ; GARNER HR ; FISHER W ; DIAZ-ARRASTIA R ; O'SUILLEABHAIN P ; ROSENBLATT KP.** Serum biomarkers for Alzheimer's disease: proteomic discovery. *Biomed Pharmacother,* 2007, vol. 61, 383
- **GOLDKNOPF IL ; SHETA EA ; BRYSON J ; FOLSOM B ; WILSON C ; DUTY J ; YEN AA ; APPEL SH.** Complement C3c and related protein biomarkers in amyotrophic lateral sclerosis and Parkinson's disease. *Biochem Biophys Res Commun,* 2006, vol. 342, 1034
- **GREWAL RP ; MORGAN TE ; FINCH CE.** ClqB and clusterin mRNA increase in association with neurodegeneration in sporadic amyotrophic lateral sclerosis. *Neurosci Lett,* 1999, vol. 271, 65
- Statistical Methods for Meta-Analysis. Academic Press, 1985
- **HOLLAND PC ; HANCOCK SW ; HODGE D ; THOMPSON D ; SHIRES S ; EVANS S.** Degradation of albumin in meningococcal sepsis. *Lancet,* 2001, vol. 357, 2102
- **HUGHES GJ ; FRUTIGER S ; PAQUET N ; RAVIER F ; PASQUALI C ; SANCHEZ JC ; JAMES R ; TISSOT JD ; BJELLQVIST B ; HOCHSTRASSER DF.** Plasma protein map: an update by microsequencing. *Electrophoresis,* 1992, vol. 13, 707
- **ILZECKA J ; STELMASIAK Z ; DOBOSZ B.** Interleukin-lbeta converting enzyme/Caspase-1 (ICE/Caspase-1) and soluble APO-1/Fas/CD 95 receptor in amyotrophic lateral sclerosis patients. *Acta Neurol Scand,* 2001, vol. 103, 255
- **JACOBS DI ; VAN RIJSSEN MS ; VAN DER HEIJDEN R ; VERPOORTE R.** Sequential solubilization of proteins precipitated with trichloroacetic acid in acetone from cultured Catharanthus roseus cells yields 52% more spots after two-dimensional electrophoresis. *Proteomics,* 2001, vol. 1, 1345
- **JACQUIER A ; BUHLER E ; SCHÄFER MK ; BOHL D ; BLANCHARD S ; BECLIN C ; HAASE G.** Alsin/Rac1 signaling controls survival and growth of spinal motoneurons. *Ann Neurol,* 2006, vol. 60, 105
- **KAISER T ; KAMAL H ; RANK A ; KOLB HJ ; HOLLER E ; GANSER A ; HERTENSTEIN B ; MISCHAK H ; WEISSINGER EM.** Proteomics applied to the clinical follow-up of patients after allogeneic hematopoietic stem cell transplantation. *Blood,* 2004, vol. 104, 340
- **KAWAKAMI T ; HOSHIDA Y ; KANAI F ; TANAKA Y ; TATEISHI K ; IKENOUE T ; OBI S ; SATO S ; TERATANI T ; SHIINA S.** Proteomic analysis of sera from hepatocellular carcinoma patients after radiofrequency ablation treatment. *Proteomics,* 2005, vol. 5, 4287
- **KIM HJ ; CHO EH ; YOO JH ; KIM PK ; SHIN JS ; KIM MR ; KIM CW.** Proteome analysis of serum from type 2 diabetics with nephropathy. *J Proteome Res,* 2007, vol. 6, 735
- **LACOMBLEZ L ; BENSIMON G ; LEIGH PN ; GUILLET P ; MEININGER V.** Dose-ranging study of riluzole in amyotrophic lateral sclerosis. Amyotrophic Lateral Sclerosis/Riluzole Study Group II. *Lancet,* 1996, vol. 347, 1425
- **LAEMMLI UK.** Cleavage of structural proteins during the assembly of the head of bacteriophage T4. *Nature,* 1970, vol. 227, 680
- **LEE JW ; NAMKOONG H ; KIM HK ; KIM S ; HWANG DW ; NA HR ; HA SA ; KIM JR ; KIM JW.** Fibrinogen gamma-A chain precursor in CSF: a candidate biomarker for Alzheimer's disease. *BMC Neurol,* 2007, vol. 7, 14
- **LI SQ ; YUN J ; XUE FB ; BAI CQ ; YANG SG ; QUE HP ; ZHAO X ; WU Z ; WANG Y ; LIU SJ.** Comparative proteome analysis of serum from acute pulmonary embolism rat model for biomarker discovery. *J Proteome Res,* 2007, vol. 6, 150
- **MILLER RG ; MITCHELL JD ; LYON M ; MOORE DH.** Riluzole for amyotrophic lateral sclerosis (ALS)/motor neuron disease (MND. *Cochrane Database Syst Rev,* 2002, vol. 2, CD001447
- **MIZUNO Y ; AMARI M ; TAKATAMA M ; AIZAWA H ; MIHARA B ; OKAMOTO K.** Transferrin localizes in Bunina bodies in amyotrophic lateral sclerosis. *Acta Neuropathol,* 2006, vol. 112, 597
- **MIZUTANI K ; OKA N ; KUSUNOKI S ; KAJI R ; KANDA M ; AKIGUCHI I ; SHIBASAKI H.** Amyotrophic lateral sclerosis with IgM antibody against gangliosides GM2 and GD2. *Intern Med,* 2003, vol. 42, 277
- **MOSER KV ; HUMPEL C.** Blood-derived serum albumin contributes to neurodegeneration via astroglial stress fiber formation. *Pharmacology,* 2007, vol. 80, 286
- **PERLUIGI M ; POON HF ; HENSLEY K ; PIERCE WM ; KLEIN JB ; CALABRESE V ; DE MARCO C ; BUTTERFIELD DA.** Proteomic analysis of 4-hydroxy-2-nonenal-modified proteins in G93A-SOD1 transgenic mice-A model of familial amyotrophic lateral sclerosis. *Free Radic Biol Med,* 2005, vol. 38, 960
- **PESTRONK A.** Motor neuropathies, motor neuron disorders, and antiglycolipid antibodies. *Muscle Nerve,* 1991, vol. 14, 927
- **ROWLAND LP.** Diagnosis of amyotrophic lateral sclerosis. *J Neurol Sci,* 1998, vol. 160, S6

- **SHAW PJ ; WILLIAMS R.** Serum and cerebrospinal fluid biochemical markers of ALS. *Amyotroph Lateral Scler Other Motor Neuron Disord,* 2000, vol. 1, S61
- **WALGREN JL ; MITCHELL MD ; WHITELEY LO ; THOMPSON DC.** Evaluation of two novel peptide safety markers for exocrine pancreatic toxicity. *Toxicol Sci,* 2007, vol. 96, 184
- **YAGAME M ; SUZUKI D ; JINDE K ; YANO N ; NAKA R ; ABE Y ; NOMOTO Y ; SAKAI H ; SUZUKI H ; OHASHI Y.** Urinary albumin fragments as a new clinical parameter for the early detection of diabetic nephropathy. *Intern Med,* 1995, vol. 34, 463
- **ZEMAN D ; ADAM P ; KALISTOVÁ H ; SOBEK O ; KELBICH P ; ANDEL J ; ANDEL M.** Transferrin in patients with multiple sclerosis: a comparison among various subgroups of multiple sclerosis patients. *Acta Neurol Scand,* 2000, vol. 101, 89